(19) Europäisches Patentamt
European Patent Office
Office européen des brevets

(11) **EP 4 692 302 A1**

(12) **EUROPEAN PATENT APPLICATION**
published in accordance with Art. 153(4) EPC

(43) Date of publication:
**11.02.2026 Bulletin 2026/07**

(21) Application number: **24777931.7**

(22) Date of filing: **22.03.2024**

(51) International Patent Classification (IPC):
*C12M 1/34* (2006.01)      *C12M 1/00* (2006.01)
*C12Q 1/02* (2006.01)      *G06V 20/69* (2022.01)

(52) Cooperative Patent Classification (CPC):
**C12M 1/00; C12M 1/34; C12M 1/42; C12Q 1/02;
G01N 11/00; G01N 21/55; G01N 33/543;
G06V 20/69**

(86) International application number:
**PCT/CN2024/083363**

(87) International publication number:
**WO 2024/199142 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 24.03.2023  CN 202310299296
24.03.2023  CN 202310299292
24.03.2023  CN 202310299290
24.03.2023  CN 202310299306
24.03.2023  CN 202310299309
24.03.2023  CN 202310297791
24.03.2023  CN 202310299308

(71) Applicant: **Yigong Ruixin (Xiamen) Technology
Co., Ltd
Xiamen, Fujian 361015 (CN)**

(72) Inventor: **LIN, Zhe
Xiamen, Fujian 361015 (CN)**

(74) Representative: **Chung, Hoi Kan
Mandarin IP Limited
7 Cherry Trees
Great Shelford
Cambridge CB22 5XA (GB)**

(54) **MULTI-MODAL BIOPHYSICAL CHARACTERIZATION APPARATUS, SYSTEM, METHOD AND USE**

(57)    The present application relates to the field of cell biology, and more specifically, to a multimodal biophysical characterization apparatus, a multimodal biophysical characterization system, a multimodal biophysical characterization method, and its use. The present application can achieve multimodal, high-resolution, high-throughput, high-sensitivity, low-cost, low-damage, and configurable physical characterization of single cells or multicellular aggregates, and can subject the sample to be tested to stimuli of different types and intensities (such as mechanical stimulation, electrical stimulation, light stimulation, etc.) to better simulate in vivo microenvironment, as well as perform operations on samples at specific positions or regions (such as marking, fixing, ablating, cutting, extracting, sorting, etc.), and combine with other characterization methods (such as protein staining, histochemical staining, single-cell sequencing, etc.) for comparative analysis. The multimodal biophysical characterization apparatus provided by the present application is applicable for fields of synthetic biology, diagnostics, drug discovery, early tumor screening, cell therapy, and precision medicine.

Figure 1

**Description**

**TECHNICAL FIELD**

The present application relates to the field of cell biology, and more specifically, to a multimodal biophysical characterization apparatus, a multimodal biophysical characterization system, a multimodal biophysical characterization method, and its use.

**BACKGROUND ART**

Cells are the basic units of life. Cell behaviors, including the processes of adhesion, migration, differentiation, and apoptosis, and their dynamic changes in different physiological and pathological processes, as well as their interactions with small and large molecule substances, are of great significance for understanding and regulating life phenomena.

The physical characteristics of cells or multicellular aggregates, such as cell force (also called cell mechanical force or cell traction force) and cell hardness (stiffness), play an important role in maintaining life activities and regulating biological functions. For example, cell force can affect processes such as cell morphology, migration, proliferation, differentiation, and signal transduction. It can also, together with other biochemical signals, play a crucial regulatory role in processes such as embryonic development, stem cell differentiation, immune processes, wound healing, and cancer metastasis, and has therefore become a target for many disease treatments. Some studies have found that there are huge differences in the maximum cell mechanical force between normal cells, benign, and malignant tumor cells. Cell hardness refers to the ability of a cell to deform under external force, and is usually used to describe the softness or hardness of a cell. Cell hardness is of great significance for the identification, evaluation, and treatment monitoring of tumors. In cancer research, cell hardness is widely used for the identification and separation of tumor cells, such as circulating tumor cells (CTCs), etc. Compared with normal cells, tumor cells usually have higher hardness. Changes in cell hardness are generally considered an important feature of tumor cell invasion and metastasis. Cell hardness can also be used to evaluate the degree of malignancy of a tumor, which helps in formulating more accurate treatment plans. At the same time, cell hardness is also at the core of the immune escape mechanism. In addition, the characterization and study of cell hardness are of great significance in the mechanisms of inflammation, the diagnosis and treatment of blood diseases, the diagnosis and monitoring of liver diseases, and drug screening and development. In short, cell hardness can objectively reflect changes in the internal structure and composition of a cell. The study of cell hardness is beneficial for solving basic biological problems and for applications in clinical diagnosis, drug discovery, early tumor screening, cell therapy, etc. Therefore, characterizing the type, state, behavior, etc., of cells and multicellular aggregates, especially the high-throughput and precise characterization of cell mechanical force and cell hardness, is an important topic in fields such as bioengineering, cell biology, and physical biology. It is necessary to perform precise, efficient, and comprehensive physical characterization of cells or multicellular aggregates of different types and states.

Currently, the main methods for characterizing cells and multicellular aggregates are as follows: such as physical methods: using instruments to measure the mechanical or electrical properties of cells, such as traction force, adhesion force, elastic modulus, electrical impedance, etc. These methods can reflect the morphology, function, and metabolic state of cells, but existing technologies usually require expensive equipment and data processing techniques. For example, Atomic Force Microscopy (AFM), Traction Force Microscopy (TFM), Electrical Impedance Spectroscopy (EIS), Microfluidic Chip (MFC), etc. However, these methods have some limitations, such as complex operation, low throughput, and high cost. They also have difficulties in long-term and real-time monitoring of cells due to limitations such as throughput or phototoxicity. There are also biochemical methods, which use reagents or markers to measure cells. These methods can sensitively detect cell status and binding with macromolecules, but may also affect their own properties or functions. For example, Fluorescence Resonance Energy Transfer (FRET), Biotin-Avidin System, Enzyme-Linked Immunosorbent Assay (ELISA), etc. Although chemical methods can sensitively detect the binding between cells and molecules, they usually require marking or modification of the target and may interfere with its normal function. Therefore, on the basis of existing technology, it is very necessary, extremely urgent, and highly valuable to propose a system and method that can monitor the physical state of cells and multicellular aggregates non-invasively and label-free, predict the differentiation and behavior of cells and multicellular aggregates, and subsequently propose a system and method for real-time, high-throughput, low-cost characterization of the cell mechanical force, cell softness/hardness, and their simulated responses to external stimuli of cells and multicellular aggregates.

**SUMMARY OF THE INVENTION**

For this purpose, it is necessary to provide a solution that can overcome the above-mentioned shortcomings of the prior art, enabling quantitative measurement and monitoring of cell mechanical force and/or cell hardness without expensive equipment, in real-time, with high throughput, and at a low cost, to meet the needs of basic biological problems, clinical diagnosis, drug discovery, early tumor screening, cell therapy, etc., for the next generation of physical characterization tools and methods for cells and multicellular aggregates.

To achieve the above objective, in a first aspect, the present application provides a multimodal biophysical characterization apparatus, comprising:

a base, and

a micropillar or a micropillar array composed of at least one micropillars arranged on the base, capable of undergoing a state change under the action of cell mechanical force and/or magnetic force,

wherein the state change includes at least one of: pulling, movement in various directions, and deformation;

wherein the micropillar comprises a bottom end connected to the base, a side surface, a pillar body enclosed by the side surface, and a top end opposite the bottom end and away from the base, wherein a light-reflecting layer is provided at any position on the top end, side surface, and/or within the pillar body of the micropillar, and a magnetic material is provided at any position on the top end, side surface, and/or within the pillar body of the micropillar, and preferably, a magnetic metal light-reflecting layer is provided on the top end of the micropillar.

The base in the present application can be any regular or irregular surface, including a flat surface, an inclined surface, a curved surface, or an irregular curved surface. In addition to customizing the three-dimensional morphology of the adhesion surface for cells or multicellular aggregates, it can provide a three-dimensional attachment environment. Optionally, the length of the micropillars on an inclined or curved surface can also be different, leading to different micropillar hardness. In the present application, the length, aspect ratio, and hardness of micropillars on the same base can also be different, which can expand the measurable range and apply different hardness stimuli to the biological entity to be tested. The magnetic material in the present application refers to materials that react in some way to a magnetic field, including but not limited to ferromagnetic materials, paramagnetic materials, diamagnetic materials, ferrimagnetic substances, antiferromagnetic substances, and superparamagnetic materials.

When the multimodal biophysical characterization apparatus provided by the present application characterizes a cell or multicellular aggregate, the micropillars can pierce the cell or multicellular aggregate. The piercing force includes but is not limited to the gravity of the cell or multicellular aggregate, the attractive adhesion produced by a substance with a mechanism for promoting cell adhesion on the cell or multicellular aggregate, applying pressure on the micropillar or the cell or multicellular aggregate, etc. In particular, after the micropillar pierces the cell or multicellular aggregate, the magnetic material at any position on the top end, side surface, or within the pillar body of the micropillar is acted upon by a magnetic force, causing the micropillar to undergo at least one of: pulling, movement in various directions, and deformation.

The term "several" in the present application does not mean a plural quantity, but refers to one or more.

Furthermore, a light-reflecting layer is provided on the top end of the micropillar, and an anti-reflection layer is provided on the side surface of the micropillar and/or the surface of the base. This arrangement can significantly improve the signal-to-noise ratio of the optical signal in biophysical characterization, making the measurement results more accurate.

Furthermore, the multimodal biophysical characterization apparatus further comprises several cell-confining structures arranged on the base, wherein the cell-confining structure comprises one or several confining surfaces, the confining surface is a plane or a curved surface, the confining surface is perpendicular to the plane where the base is located, connected to the base or integrally formed with the base, and the height of the confining surface is greater than that of the micropillars and encloses a preset number of micropillars.

Furthermore, at least one of the base, micropillars, and confining surfaces is provided with a substance that interacts with cells, wherein the substance that interacts with cells comprises:

\at least one of a substance that promotes cell adhesion, a substance that interacts with cell surface receptors, a biologically active substance, and a biologically inactivated substance.

Preferably, the substance that promotes cell adhesion may include, but is not limited to, at least one of the following substances: (1) extracellular matrix molecules, including but not limited to collagen, fibronectin, vitronectin, laminin, or proelastin; (2) mimics of the extracellular matrix, including but not limited to polypeptides containing the RGD adhesion sequence; (3) substances with a cell adhesion-promoting mechanism, including but not limited to polylysine; substances that interact with cell surface receptors include but are not limited to antibodies.

Furthermore, at least one of the base, micropillars, and confining surfaces is provided with a substance that has anti-cell adhesion properties. This arrangement can further define the adhesion range of the cell or multicellular aggregate, ensuring it falls within a predetermined area to be tested, and can be used to control the morphology of the cell, including its size, shape, and tropism, and to better regulate the adhesion, migration, and differentiation of the cell or multicellular aggregate.

Furthermore, the substance that interacts with cells is provided on the top end of the micropillars, and multiple micropillars provided with the substance that interacts with cells form a first preset pattern; and/or the substance with anti-cell adhesion properties is provided on the top end of the micropillars, and multiple micropillars provided with the substance with anti-cell adhesion properties form a second preset pattern. The first preset pattern may be the same as or different from the second preset pattern; this naming in the present application is merely for distinguishing and identifying the patterns formed by the entirety of micropillars provided with the substance that interacts with cells and the substance with anti-cell adhesion properties, respectively.

Furthermore, a fluorescent substance is provided on the extension part of the micropillar from the top end to the bottom end. This extension part includes the side surface of the micropillar and the solid part of the pillar body enclosed by the side surface. This arrangement can achieve the purpose of more intuitively calibrating the cell or multicellular aggregate to amplify the signal and improve the signal-to-noise ratio. At the same time, by staining the cell or multicellular aggregate to be characterized with a fluorescent substance of another color, the hardness of the cell or multicellular aggregate can be judged by the depth of piercing, enhancing the characterization signal.

Furthermore, the base and/or micropillars are made of a conductive material. The purpose of using a conductive material for the base and/or micropillars is, in addition to being able to apply electrical stimulation during the process of characterizing the cell or multicellular aggregate, also to achieve the purpose of receiving electrical signals from the cell.

Specifically, the multicellular aggregate of the present application is combined with the multimodal biophysical characterization apparatus in various ways, two specific ways of which are, for example:

The first way of combination: a culture medium is placed on the micropillars of the multimodal biophysical characterization apparatus, and cells are transplanted into the culture medium on the micropillars to be cultured into a multicellular aggregate. In some other embodiments, with this way of combination, under the condition that cell mechanical force information is output in a visual form, the cultivation process of the cells can be monitored in real-time, to be applied to studying the effects of chemical, biological, and physical external stimuli such as culture media, drugs, etc., on cell growth.

The second way of combination: the already-cultured multicellular aggregate is directly adhered to the micropillars of the multimodal biophysical characterization apparatus for detection.

To achieve the above objective, in a second aspect, the present application provides a multimodal biophysical characterization system, comprising:

at least one multimodal biophysical characterization apparatus according to the first aspect of the present application;

a light signal emitting device, wherein the light signal emitting device is used to emit a predetermined light beam;

a light signal detecting device, wherein the light signal detecting device is used to detect the light beam reflected from the light-reflecting layer;
and

a magnetic field emitting device, wherein the magnetic field emitting device is used to emit a magnetic field to generate a magnetic force with the magnetic material;

wherein the light beam emitted by the light signal emitting device illuminates the light-reflecting layer through an incident light path, and the light beam reflected by the light-reflecting layer enters the light signal detecting device through a reflected light path.

Besides the light signal emitting device, light signal detecting device, and magnetic field emitting device, the multimodal biophysical characterization system of the present application can be constituted by using only one multimodal biophysical characterization apparatus according to the first aspect of the present application to measure the cell mechanical force and its single-sided hardness of a cell or multicellular aggregate, or by using two or more multimodal biophysical characterization apparatuses according to the first aspect of the present application to measure the cell mechanical force and its double-sided or multi-sided hardness of a cell or multicellular aggregate.

Furthermore, the multimodal biophysical characterization system further comprises a light signal analysis device, wherein the light signal analysis device is used to analyze the light signal detected and acquired by the light signal detecting device.

Furthermore, the multimodal biophysical characterization system further comprises a data processing device, wherein the data processing device is used to compute and process the light signal to obtain the results (e.g., including but not limited to numerical magnitude, directionality, and trend of change) and spatial distribution of cell mechanical force and/or cell softness/hardness. Furthermore, the data processing device can also provide predictive information on cell behavior and differentiation direction based on the results and spatial distribution of cell mechanical force and/or cell softness/hardness.

Furthermore, the multimodal biophysical characterization system further comprises a pressure transmitting device, wherein the pressure transmitting device is used to apply pressure to the cells. The pressure transmitting device includes but is not limited to a gravity block, an automated pressure device, a probe, etc. The pressure transmitting device may include a pressure sensor to simultaneously sense the magnitude of the applied pressure to control an appropriate pressure value.

Furthermore, the light signal emitting device has a light source, the light signal emitting device has a light source, the light source comprising a first light source arranged at the bottom of the base and/or a second light source arranged at the side of the base and/or a third light source arranged at the top end opposite the bottom end and away from the base, wherein the light beam emitted by the first light source and/or second light source and/or third light source reaches the micropillars.

In the present application, the first light source, second light source, and third light source do not imply a limitation on the setting order and type of light sources, but are merely for distinguishing light sources at different positions. Preferably, the second light source employs a waveguide illumination source.

Furthermore, the multimodal biophysical characterization system further comprises a device for causing motion or deformation, wherein the device for causing motion or deformation is used to apply a mechanical force to the multimodal biophysical characterization system by causing deformation of the multimodal biophysical characterization system. Preferably, the device for causing motion or deformation includes but is not limited to a pulling device and/or an inflation/deflation device, wherein the pulling device is used to apply an appropriate mechanical pulling force to the base to control the stretching motion or deformation of the base in the horizontal direction, and the inflation/deflation device is used to form a motion or deformation on a curved surface of the base.

Furthermore, the multimodal biophysical characterization system is constituted by two or more of the multimodal biophysical characterization apparatuses to form a double-sided structure or a multi-sided structure, wherein the outside of the double-sided structure or multi-sided structure is the base, and the inside encloses a three-dimensional accommodation cavity for cells. This arrangement can provide the cell with a nearly three-dimensional attachment environment, and more accurately characterize the multi-faceted, diverse physical properties of the cell or cell multimer within a three-dimensional scale, being more precise and closer to the in vivo environment.

Furthermore, when a double-sided or multi-sided structure is constituted by 2 or more of the multimodal biophysical characterization apparatuses, the cell-confining structures located on different multimodal biophysical characterization apparatuses are adapted to each other.

In a third aspect, the present application provides a method for performing physical characterization of cells using the multimodal biophysical characterization system according to the second aspect of the present application, comprising the following steps:

emitting a predetermined light beam with the light signal emitting device;

detecting the light beam after it has interacted with the multimodal biophysical characterization apparatus with the light signal detecting device.

The method provided by the present application, by using micropillars provided with a magnetic material and a light-reflecting layer substance (a fluorescent substance, an anti-light reflection layer substance, a magnetic metal light-reflecting layer, one or more of which can also be provided), controlling the spacing between micropillars, and coating substances with a mechanism for promoting cell adhesion, such as antibodies or extracellular matrix, or with a mechanism for anti-cell adhesion, such as F127, at specific locations, can autonomously switch between measuring cell mechanical force or cell rigidity, or simultaneously measure cell mechanical force and cell hardness as needed. It can achieve simultaneous measurement of double-sided or multi-sided cell mechanical force, and can also achieve measurement of the cell hardness at a specific position of a certain cell or multicellular aggregate, as well as simultaneous measurement of cell mechanical force and cell hardness. It is the first time that the active mechanical force of a cell within a cell's space is combined with the features of cell softness/hardness.

Furthermore, the method further comprises the step of emitting a magnetic field of a predetermined direction and intensity with the magnetic field emitting device,

optionally, before, during, or after the physical characterization, further comprising the step: applying stimuli of different types and intensities to the sample to be characterized;

optionally, the stimulus comprise at least one of a physical stimulus, a chemical stimulus, and a biological stimulus;

optionally, the physical stimulus, chemical stimulus, and biological stimulus is selected from a group consisted of:

drugs, mechanical force, hardness, biochemistry, electric field, flow field, tropic guidance, radiation.

In particular, before, during, or after the physical characterization of the cell or multicellular aggregate, operations including but not limited to marking, fixing, ablating, cutting, extracting, sorting, etc., can be performed on the sample to be tested at specific positions or regions, and comparative analysis can be performed in conjunction with other characterization methods including but not limited to protein staining, histochemical staining, single-cell sequencing, etc.

Furthermore, the method further comprises a light signal analysis step: using the light signal analysis device to perform a comparative analysis of the reflected light beams before and after a fluid flows through the multimodal biophysical characterization apparatus. Within a certain range of deformation, the deformation and the attenuation of the light reflection signal are linearly related. The state of the fluid can be inferred from the strength of the light reflection signal generated by the micropillar deformation, to achieve fluid flow rate calibration; and/or, using the light signal analysis device to perform a comparative analysis of the reflected light beams before and after the magnetic field emitting device emits a magnetic field of a predetermined direction and intensity, to obtain information on the cell mechanical force and/or cell hardness of the sample to be characterized;

wherein the cell mechanical force information includes the magnitude, direction, frequency, distribution, and dynamic changes over time of the cell mechanical force at a specific position of a cell or multicellular aggregate, and the cell hardness information includes the magnitude of hardness, spatial distribution, and dynamic changes over time of different layers at a specific position within a cell or multicellular aggregate.

In a fourth aspect, the present application provides the use of the multimodal biophysical characterization apparatus as described in the first aspect in the fields of synthetic biology, cell therapy, drug discovery, characterization of cell-cell interactions, early tumor screening, or precision medicine, wherein, preferably, the use comprises: determining or predicting whether transfection is successful; determining or predicting whether the sample to be characterized is a high-expression cell for a certain bioactive substance; typing normal or pathological cells; determining or predicting the state and differentiation direction of cells; typing for sensitivity to drugs and treatments; predicting the behavior of cells, including but not limited to state, differentiation, proliferation, migration, and apoptosis; determining or predicting the interaction between a cell or multicellular aggregate and a bioactive substance, and the interaction between (multi)cell-(multi)cell, by obtaining at least one of the magnitude, spatial distribution, and dynamic change trend of cell mechanical force and cell hardness through the multimodal biophysical characterization apparatus.

Distinguished from the prior art, the above technical solution has the following advantages:

(1) High throughput and low cost: Compared with existing AFM, TFM, MFC, and ordinary micropillar arrays, the technical solution of the present application gets rid of the dependence on expensive equipment, greatly simplifies the operation process, and does not require high-resolution confocal microscopy imaging. High-throughput monitoring of cells can be achieved simply by monitoring the intensity of reflected light, and the cost is low.

(2) Capable of multimodal biophysical characterization: A magnetic metal reflective layer and magnetic material are added to the top end of the micropillar. By changing parameters such as the coating composition characteristics, spacing, and movement logic of the micropillar, it is possible to switch between measuring cell mechanical force or cell hardness, or to measure both simultaneously, achieving a more flexible and precise comprehensive characterization of the physical features of cells.

(3) Dynamically and in real-time changeable micropillar properties, such as softness/hardness, movement mode, etc., to dynamically and in real-time optimize the measurement range, and configurable with multiple conditions for different stimuli: By adding components such as magnetic materials, light sources, electrodes, pressure transmitters, etc., the micropillars can be dynamically controlled and adjusted to adapt to different measurement needs and conditions. At the same time, physical or chemical stimuli can be applied to the cell or multicellular aggregate to observe the effects of different conditions and stimuli on the physical features of the cell or multicellular aggregate.

(4) Capable of implementing multiple operation modes: By setting up devices such as cell confinement devices, sandwich structures, etc., operations such as morphological control, pressure application, position marking, sample extraction, etc., can be performed on the cell or multicellular aggregate, and can be combined with other characterization methods (protein staining, histochemical staining, single-cell sequencing, etc.) for further comparative analysis.

(5) High resolution, high sensitivity, suitable for long-term monitoring: Real-time monitoring of single cells and multicellular aggregates can be performed with high resolution. The micropillar deformation signal is amplified simply through the light reflection signal, increasing the sensitivity of deformation monitoring. Real-time monitoring: No fluorescence is required, which avoids the phototoxic effects of lasers on cells or multicellular aggregates, making it suitable for long-term monitoring and for studying the long-term response of cells or multicellular aggregates to drugs.

(6) Configurable: The composition and function of the system can be flexibly configured according to the usage scenario. For example, using a magnetic field in the following three scenarios can achieve different effects: first, by driving the micropillar to deform and move with a magnetic field to measure cell hardness, or by applying a pulling force parallel to the micropillar, which is equivalent to increasing the hardness of the micropillar, to adjust and optimize the measurement range of the micropillar and/or to give the cell mechanical or motion stimulation in real-time.

In summary, the present application can achieve multimodal, high-resolution, high-throughput, high-sensitivity, low-cost, low-damage, and configurable physical characterization of single cells or multicellular aggregates, and can subject the sample to be tested to stimuli of different types and intensities (such as mechanical stimulation, electrical stimulation, light stimulation, etc.) to better simulate the in vivo microenvironment, as well as perform operations on samples at specific positions or regions (such as marking, fixing, ablating, cutting, extracting, sorting, etc.), and combine with other characterization methods (such as protein staining, histochemical staining, single-cell sequencing, etc.) for comparative analysis. The multimodal biophysical characterization apparatus provided by the present application is suitable for fields such as synthetic biology, diagnostics, drug discovery, early tumor screening, cell therapy, and precision medicine.

**BRIEF DESCRIPTION OF THE DRAWINGS**

Figure 1 is a schematic diagram of a multimodal biophysical characterization apparatus in specific embodiment 1 of the present application;

Figure 2 is a schematic diagram in the A-A' direction of a specific embodiment of the present application (light-reflecting layer provided on the top end of the micropillar);

Figure 3 is a schematic diagram in the A-A' direction of a specific embodiment of the present application (light-reflecting layer provided on the side surface of the micropillar);

Figure 4 is a schematic diagram in the A-A' direction of a specific embodiment of the present application (light-reflecting layer provided at any position within the pillar body of the micropillar);

Figure 5 is a schematic diagram in the A-A' direction of a specific embodiment of the present application (light-reflecting layer provided on the top end and side surface of the micropillar);

Figure 6 is a schematic diagram in the A-A' direction of a specific embodiment of the present application (light-reflecting layer provided at any position within the pillar body and on the side surface of the micropillar);

Figure 7 is a schematic diagram in the A-A' direction of a specific embodiment of the present application (magnetic metal light-reflecting layer provided on the top end of the micropillar);

Figure 8 is a schematic diagram of an anti-reflection layer provided on the side surface of the micropillar in a specific embodiment of the present application;

Figure 9 is a schematic diagram of an anti-reflection layer provided on the surface of the base in a specific embodiment of the present application;

Figure 10 is a schematic diagram of an anti-reflection layer provided on the side surface of the micropillar and the surface of the base in a specific embodiment of the present application;

Figure 11 is a schematic diagram of a magnetic material provided on the side surface of the micropillar in a specific embodiment of the present application;

Figure 12 is a schematic diagram of a magnetic material provided on the upper part of the light-reflecting layer at the top end of the micropillar in a specific embodiment of the present application;

Figure 13 is a schematic diagram of a magnetic material provided at any position within the pillar body of the micropillar in a specific embodiment of the present application;

Figure 14 is a schematic diagram of a magnetic material provided extending from the top end to the bottom end of the micropillar and located on the upper part of the light-reflecting layer in a specific embodiment of the present application;

Figure 15 is a schematic diagram of a magnetic material provided extending from the top end to the bottom end of the micropillar and located on the upper part of the light-reflecting layer, with an anti-reflection layer provided on the surface of the base, in a specific embodiment of the present application;

Figure 16 is a schematic diagram of a magnetic material provided within the pillar body of the micropillar and located on the upper part of the light-reflecting layer, with an anti-reflection layer provided on the surface of the base, in a specific embodiment of the present application;

Figure 17 is a schematic diagram of measuring a single cell with a multimodal biophysical characterization apparatus having a confining structure in a specific embodiment of the present application;

Figure 18 is a schematic diagram of measuring a single cell with a multimodal biophysical characterization apparatus having a confining structure and a cell adhesion-promoting substance provided on the surface of the base in a specific embodiment of the present application;

Figure 19 is a schematic diagram of measuring a single cell with a multimodal biophysical characterization apparatus having a confining structure, a cell adhesion-promoting substance provided on the surface of the base, and an anti-cell adhesion substance provided on the confining surface in a specific embodiment of the present application;

Figure 20 is a schematic diagram of a fluorescent substance provided within the pillar body of the micropillar (below the top end) and located below the light-reflecting layer in the present application;

Figure 21 is a schematic diagram of a fluorescent substance provided on the top end of the micropillar and composited with the light-reflecting layer material in the present application;

Figure 22 is a schematic diagram of a fluorescent substance provided on the side surface of the micropillar in the present application;

Figure 23 is a schematic diagram of a fluorescent substance provided at any position within the pillar body of the micropillar in the present application;

Figure 24 is a schematic diagram of measuring the double-sided hardness of a single cell using a double-sided structure composed of 2 multimodal biophysical characterization apparatuses in a specific embodiment of the present application;

Figure 25 is a schematic diagram of measuring the multi-sided hardness of a single cell using a multi-sided structure composed of 3 multimodal biophysical characterization apparatuses in a specific embodiment of the present application;

Figure 26 is a schematic diagram of a measurement where the cell-confining structures of a double-sided structure composed of 2 multimodal biophysical characterization apparatuses are adapted to each other in a specific embodiment of the present application;

Figure 27 is a schematic diagram of a multimodal biophysical characterization system (light source provided below the base) in a specific embodiment of the present application;

Figure 28 is a schematic diagram of a multimodal biophysical characterization system (light source provided at the side and/or above the base) in a specific embodiment of the present application;

Figure 29 shows an example of measuring a multicellular aggregate using a double-sided structure composed of the multimodal biophysical characterization apparatus provided by the present application;

Figure 30 shows a schematic diagram of the preparation method, electron microscope characterization results, and mechanical calculation principle of the multimodal biophysical characterization apparatus;

Figure 31 shows the method and experimental results of calibrating the multimodal biophysical characterization apparatus using a flow field (micropillar deflection and light reflection signal attenuation are linearly related within a certain range);

Figure 32 shows the experimental results of simultaneously characterizing the mechanical force and hardness of tumor cells with the multimodal biophysical characterization apparatus (a correlation exists between cell mechanical force and hardness);

Figure 33 shows the experimental results of using the multimodal biophysical characterization apparatus to measure hardness with a magnetic field and cross-validating with microscope Z-scanning in a specific embodiment of the present application;

Figure 34 shows the experimental results of characterizing tumor tissue and drug testing under multimodal biophysics in a specific embodiment of the present application;

Figure 35 shows the experimental results of characterizing immune cell activation and validation against a gold standard under multimodal biophysics in a specific embodiment of the present application.

## DESCRIPTION OF REFERENCE NUMERALS

1, multimodal biophysical characterization apparatus; 11, base; 12, micropillar; 120, bottom end; 121, side surface; 122, pillar body; 123, top end; 13, light-reflecting layer; 131, magnetic material; 132, magnetic metal light-reflecting layer; 14, anti-reflection layer; 15, confining surface; 16, substance that interacts with cells; 17, substance with anti-cell adhesion properties; 18, fluorescent substance.

## DETAILED DESCRIPTION OF EMBODIMENTS

To explain in detail the technical content, structural features, achieved objectives, and effects of the technical solutions, the following will be described in detail in conjunction with specific embodiments and accompanying drawings.

The mention of "an embodiment" in this text means that a specific feature, structure, or characteristic described in conjunction with the embodiment can be included in at least one embodiment of the present application. The appearance of the phrase "an embodiment" in various places in the specification does not necessarily all refer to the same embodiment, nor does it specifically limit its independence or association with other embodiments. In principle, in the present application, as long as there is no technical contradiction or conflict, the various technical features mentioned in each embodiment can be combined in any way to form a corresponding implementable technical solution.

In the description of the present application, the term "and/or" is an expression used to describe the logical relationship between objects, indicating that three relationships can exist. For example, A and/or B means: the existence of A, the existence of B, and the simultaneous existence of A and B. In addition, the character "/" in this text generally indicates that the associated objects before and after have an "or" logical relationship.

In the present application, terms such as "first" and "second" are only used to distinguish one entity or operation from another, and do not necessarily require or imply any actual quantity, priority, or order between these entities or operations.

Without further limitations, in the present application, the use of expressions such as "comprising," "including," "having," or other similar expressions is intended to cover a non-exclusive inclusion. These expressions do not exclude the possibility that other elements may also exist in the process, method, or product that includes the elements, such that a process, method, or product that includes a series of elements may include not only those defined elements, but also other elements not explicitly listed, or elements inherent to such a process, method, or product.

Consistent with the understanding in the "Examination Guidelines," in the present application, expressions such as "greater than," "less than," "exceeding," etc., are understood as not including the number itself; expressions such as "above," "below," "within," etc., are understood as including the number itself. Furthermore, in the description of the embodiments of the present application, the meaning of "multiple" is two or more (including two), and similar expressions related to "multi-," such as "multi-group," "multi-time," etc., are to be understood in this way, unless otherwise explicitly and specifically limited.

Cell Traction Force, also called cell mechanical force, refers to the force that a cell exerts on its surrounding environment through its cytoskeleton and adhesion structures. This force can cause deformation and displacement between the cell and the external matrix, thereby affecting biological processes such as cell morphology, movement, signal transduction, and gene expression. During tumor development, changes in cell mechanical force are associated with many key biological processes. Therefore, studying cell mechanical force is of great significance in identifying tumors, specifically as follows:

Migration and metastasis of tumor cells: Tumor cells control their movement and migration in the external matrix by regulating mechanical force. Changes in mechanical force can lead to changes in cell morphology and movement patterns, thereby affecting the metastasis and invasion of tumor cells.

Tumor microenvironment: The cell-matrix interaction in the tumor microenvironment is regulated by mechanical force, which can affect key biological processes such as cell growth, proliferation, angiogenesis, and immune escape, thereby affecting tumor growth and metastasis.

Tumor therapy: The mechanical force of tumor cells is related to their sensitivity and resistance to chemotherapy and radiotherapy. Therefore, studying cell mechanical force can provide new ideas and methods for the personalized and targeted therapy of tumors.

In summary, as an important biological indicator in the process of tumor development, cell mechanical force can help us to deeply understand the development mechanism of tumors, identify tumors, and provide new ideas and methods for the prevention, treatment, and personalized medicine of tumors.

Besides tumors, the characterization and study of cell mechanical force are also of great significance in many other diseases and biological processes, specifically as follows:

Cell migration and development: Cell mechanical force is an important factor controlling cell migration and tissue development, and plays a key role in cell migration and morphological changes in biological processes such as embryonic development, tissue regeneration, and repair.

Nervous system: The mechanical force between neurons and glial cells can affect the growth and connection of neurons, playing an important role in the normal development and functional maintenance of the nervous system, and is also related to some nervous system diseases such as spinal cord injury and neurodegenerative diseases.

Immune system: The mechanical force of immune cells such as lymphocytes, monocytes, and macrophages can affect their migration in the body and their recognition and clearance of pathogens, playing an important role in the normal functional maintenance of the immune system and in combating diseases.

In summary, the characterization and study of cell mechanical force are not only of great significance for the identification and treatment of tumors, but also play a key role in many other biological processes and diseases, helping us to deeply understand the mechanisms of biological processes and the occurrence and development of diseases, and providing new ideas and methods for the treatment and prevention of related diseases.

Currently, the tools for studying cell mechanical force can be mainly divided into three categories: microfluidic technology, microscopy technology, and micro-nano chips. For example, Traction Force Microscopy (TFM) uses an optical microscope to observe cell morphology and movement and calculates the magnitude and direction of cell mechanical force by measuring the displacement of the matrix around the cells. Another example is using a confocal microscope to capture the relative displacement caused by the deformation of micro-nano structures in the Z-axis direction to calculate the force at that point. However, existing measurement techniques require special experimental conditions and equipment, are highly dependent on microscopes, are relatively complex and expensive to construct and operate, and require certain experimental experience and technical skills. In addition, image data needs to be analyzed and processed to obtain the magnitude and direction of cell mechanical force. This process requires a series of mathematical operations and image processing, requiring high data processing skills and computational capabilities. Thirdly, techniques like traction force microscopy require placing special markers, such as microbeads or fluorescent markers, around the cells. The presence of these markers may affect the behavior of the cells, such as their growth, differentiation, and migration, thereby affecting the reliability of the measurement results. More importantly, existing measurement methods have the disadvantages of low throughput and high cost, and at the same time, the lack of sufficient time leads to low resolution. Most studies only measure static or stationary cells. For the changes in mechanical force during cell movement or deformation, current technology still has difficulty performing real-time, quantitative, and long-term monitoring and analysis.

Cell hardness (Rigidity), also called cell stiffness, refers to the ability of a cell to deform under external force, and is usually used to describe the softness or hardness of a cell. Cell hardness is of great significance for the identification, evaluation, and treatment monitoring of tumors. In cancer research, cell hardness is widely used for the identification and separation of tumor cells, such as circulating tumor cells (CTCs), etc. Compared with normal cells, tumor cells usually have higher hardness. This is because the cytoskeleton, nucleus, and intercellular matrix of tumor cells are abnormal, and the expression and distribution of cytoskeletal proteins have also changed. The high hardness of tumor cells makes it easier for them to pass through the extracellular matrix, enter the circulatory system, and migrate to other parts to form metastases. Therefore, changes in hardness are considered an important feature of tumor cell invasion and metastasis. Cell hardness can also be used to evaluate the degree of malignancy of a tumor, which helps in formulating more accurate treatment plans. Moreover, cell hardness is also at the core of the immune escape mechanism. Therefore, using cell hardness as an identification indicator for tumor cells can effectively distinguish between tumor cells and normal cells, and play an important role in the early diagnosis and treatment of tumors. Currently, some detection methods based on cell hardness have been developed, such as atomic force microscopy, cell microfluidic chips, etc. After treatment, the hardness of cells may also change, so it can also be used to monitor the treatment effect. The following are several aspects where cell hardness may be applied in different stages of tumor development:

Diagnosis: By measuring cell hardness, tumor cells and normal cells can be distinguished. Tumor cells are usually harder than normal cells, so cell hardness can be used as a diagnostic tool for the early diagnosis and differential diagnosis of tumors.

Prognosis: The hardness of tumor cells is related to their degree of malignancy. Studies have shown that tumor cells with higher hardness are more invasive and metastatic. Therefore, by measuring cell hardness, the prognosis of the tumor can be predicted, providing a basis for the selection of treatment plans.

Treatment: Cell hardness can be used as a monitoring indicator for tumor treatment. Some treatment methods can lead to changes in the hardness of tumor cells. Therefore, by measuring the changes in cell hardness, the treatment effect can be monitored, and the treatment plan can be adjusted.

New drug screening: Cell hardness can also be used for screening new drugs. Some drugs can affect the hardness of cells, so by measuring the changes in cell hardness, the effectiveness and safety of drugs can be evaluated.

Besides tumors, the characterization and study of cell hardness have many other applications and significances in biology and medicine, for example:

Inflammation: Inflammation is a response of cells and tissues. Inflammation leads to the destruction of the cell membrane and changes in permeability, which will affect the hardness of the cell. Therefore, measuring cell hardness can be used to study the mechanisms of inflammation.

Blood diseases: For example, diseases such as reduced deformability of red blood cells, anemia, etc. These diseases are usually related to the deformability and hardness of red blood cells. Therefore, measuring the hardness of red blood cells can be used for the diagnosis and treatment of these diseases.

Liver diseases: The hardness of liver tissue can be used to diagnose and monitor liver diseases, such as cirrhosis and liver fibrosis. These diseases cause the liver tissue to harden and lose its elasticity, thereby affecting the function of the liver.

Cell movement and migration: Cell hardness is related to the movement and migration of cells. Therefore, measuring the hardness of cells can be used to study the mechanisms of cell movement and migration, such as the metastasis of cancer cells and the migration of inflammatory cells.

Drug screening and development: The efficacy and toxicity of drugs may affect the hardness of cells. Therefore, measuring the hardness of cells can be used for drug screening and development to evaluate the effectiveness and toxicity of drugs. In short, cell hardness has a wide range of applications in biology and medicine, and can be used to study a variety of physiological and pathological processes and diseases, and provide important information for the diagnosis and treatment of diseases.

In the present application, "multimodal" refers to multiple modes of physical information such as cell force (cell traction force or cell mechanical force) or cell hardness (rigidity), or both cell mechanical force and cell hardness.

In the present application, "multicellular aggregate" refers to a population of cells formed by the aggregation of two or more cells, i.e., a multicellular aggregate; multicellular aggregates include cell groups obtained from in vitro or in vivo culture, such as tumor multimers, for example, including but not limited to tumor spheroids, organoids, and living tissues. Generally, the "cell" referred to in the present application includes single cells and multicellular aggregates formed by the aggregation of two or more cells.

**Embodiment 1**

**A Multimodal Biophysical Characterization Apparatus, System, and Measurement Method**

This embodiment provides a multimodal biophysical characterization apparatus that utilizes a light-reflecting layer, a magnetic material, or a magnetic metal light-reflecting layer to affect the intensity of the reflected light signal, thereby achieving the measurement of multimodal biophysical information of cells.

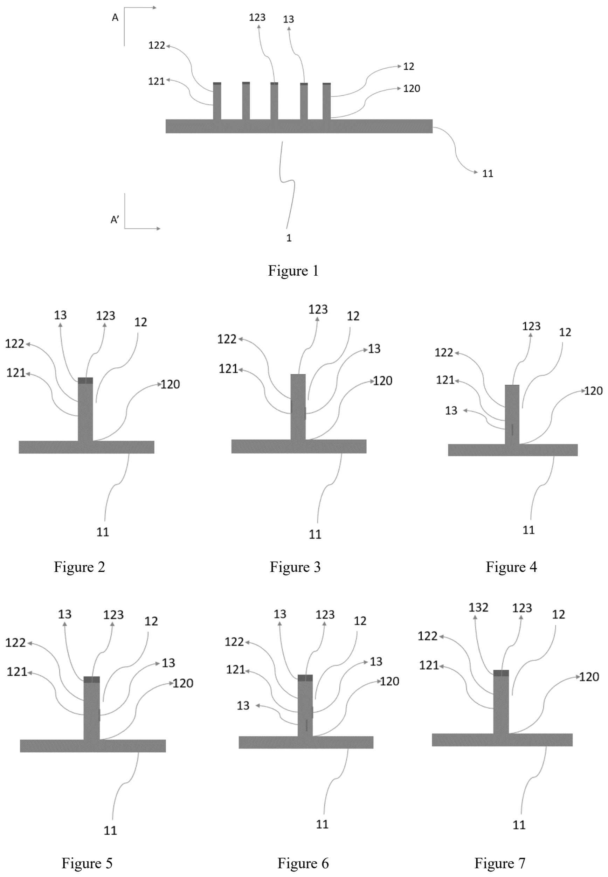

Please refer to Figure 1, which shows a multimodal biophysical characterization apparatus 1, composed of a base 11 and multiple micropillars 12 arranged on the base 11 to form a micropillar array, thus constituting the multimodal biophysical characterization apparatus 1, i.e., a micropillar chip. The micropillar 12 is composed of a bottom end 120 connected to the base 11, a side surface 121, a pillar body 122 enclosed by the side surface, and a top end 123 opposite the bottom end 120 and away from the base 11. Specifically, viewed from the A-A' direction of the multimodal biophysical characterization apparatus 1 shown in Figure 1, the top end 123 of the micropillar 12 of the present application has a coating 13 that reflects light (Figure 2); alternatively, the side surface 121 of the micropillar 12 has a coating 13 that reflects light (Figure 3); or any position within the pillar body of the micropillar has a coating that reflects light (Figure 4); or, both the top end 123 and the side surface 121 of the micropillar 12 have a coating that reflects light (Figure 5); or still, any position on the top end 123, side surface 121, and within the pillar body 122 of the micropillar 12 has a coating that reflects light (Figure 6). The top end 123 of the micropillar 12 has a coating of a magnetic metal (such as iron, cobalt, nickel, etc.) (in other embodiments, other magnetic materials can be used instead, and the position where the magnetic material is set can be the side surface or within the pillar body of the micropillar, as long as the micropillar can generate a magnetic force under a magnetic field). This coating with the magnetic metal 131 can serve as the light-reflecting layer 13. In a preferred embodiment, what is provided on the top end 123 of the micropillar 12 is a magnetic metal light-reflecting layer 13 (Figure 7).

It should be noted that the term "coating" is used in this embodiment only to indicate that the light-reflecting layer 13 in this embodiment can be prepared by a coating process, but this does not limit the light-reflecting layer 13 to being prepared only by a coating process; for example, it can also be prepared by processes such as sputtering or vapor deposition.

A multimodal biophysical characterization system is composed of one of the above multimodal biophysical characterization apparatuses 1, a light signal emitting device, a light signal detecting device, and a magnetic field emitting device. Among them, the light signal emitting device is used to emit a predetermined light beam, the light signal detecting device is used to detect the light beam reflected from the light-reflecting layer 13, and the magnetic field emitting device is used to emit a magnetic field to generate a magnetic force with the magnetic metal 131. The light beam emitted by the light signal emitting device illuminates the light-reflecting layer 13 through an incident light path, and the light beam reflected by the light-reflecting layer 13 enters the light signal detecting device through a reflected light path.

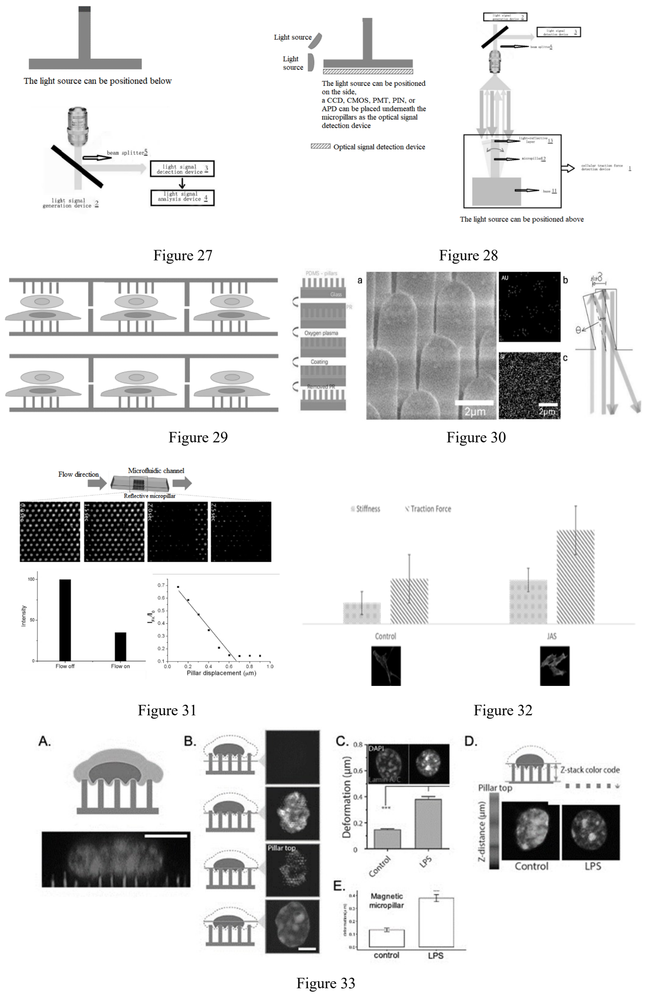

When using the above multimodal biophysical characterization system (Figures 27 and 28) to measure the multimodal biophysical characteristics of cells, the specific operating steps are as follows:

S1. Culture the cells (or cell multimers, or both single cells and cell multimers simultaneously) to be tested (characterized) on the multimodal biophysical characterization apparatus shown in Figure 1. After one to two days of cultivation, the cells to be tested (characterized) are fully attached and growing on the multimodal biophysical characterization apparatus.

S2. Emit a predetermined light beam with the light signal emitting device.

S3. Detect the light beam after it has interacted with the multimodal biophysical characterization apparatus with the light signal detecting device. The "interaction" in this embodiment may only be the reflection effect on the predetermined light beam produced by the light-reflecting layer provided at any one part of the top end or side surface of the micropillars of the multimodal biophysical characterization apparatus, or it may be the reflection effect on the predetermined light beam produced by the light-reflecting layers provided on both the top end and the side surface of the micropillars. Preferably, it may also be that, under the action of a magnetic force from a magnetic field of a specific direction and intensity emitted by the magnetic field emitting device, the magnetic metal light-reflecting layer provided on the top end of the micropillar causes the micropillar to tilt in one direction, pierce the cell to be characterized, deform, be pulled, or swing, while the magnetic metal light-reflecting layer produces a reflection effect on the light beam during the deformation process of the micropillar. Therefore, using the system of this embodiment, the mechanical force of the cell to be characterized can be measured alone, or the hardness of the cell to be characterized can be measured alone, or the mechanical force and hardness of the cell to be characterized can be measured jointly.

It should be pointed out that the cell to be characterized in this embodiment can be a single cell or a multicellular aggregate, and may also be both single cells and multicellular aggregates.

**Embodiment 2**

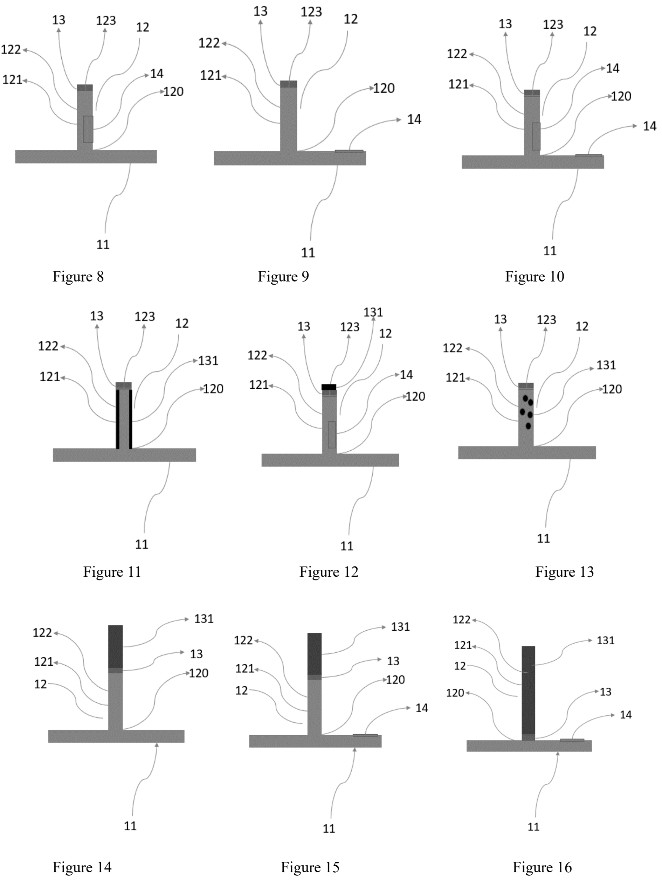

Please refer to the multimodal biophysical characterization apparatus shown in Figures 8, 9, and 10 (A-A' direction view). It differs from Embodiment 1 in that a micropillar array is formed by a base 11 and multiple micropillars 12 arranged on the base 11, thus constituting the multimodal biophysical characterization apparatus 1. The micropillar 12 is composed of a bottom end 120 connected to the base 11, a side surface 121, a pillar body 122 enclosed by the side surface, and a top end 123 opposite the bottom end 120 and away from the base 11. The top end 123 of the micropillar 12 has a magnetic metal light-reflecting layer 13 that has a light-reflecting effect. An anti-reflection layer 14 is provided on the side surface of the micropillar (Figure 8), or an anti-reflection layer 14 is provided on the surface of the base (Figure 9), or, both the side surface 121 of the micropillar 12 and the surface of the base have an anti-reflection layer 14 (Figure 10).

In other different embodiments, a light-reflecting layer 13 is provided on at least one of the top end and side surface of the micropillar, a magnetic material 131 is provided at any position on the top end, side surface, and within the pillar body of the micropillar, and various combinations can be made with at least one of the side surface and base surface of the micropillar having an anti-reflection layer 14 (Figures 11, 12, 13, 14, 15, and 16), to achieve the purpose of making the micropillar or certain specific parts of the micropillar have the ability to reflect light and generate magnetic force.

## Embodiment 3

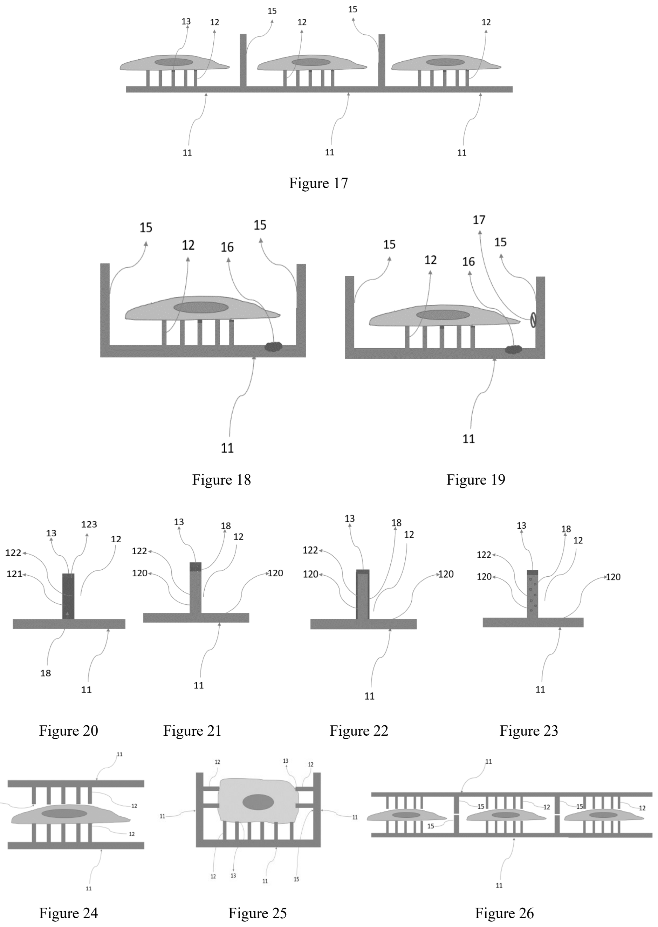

Please refer to Figure 17. The difference from Embodiments 1 and 2 is that the multimodal biophysical characterization apparatus of this embodiment further comprises a cell-confining structure. The cell-confining structure includes one or several confining surfaces 15. The confining surface 15 is a plane or a curved surface that is perpendicular to the plane where the base 11 is located, connected to the base 11 or integrally formed with the base 11, and the height of the confining surface 15 is greater than that of the micropillars 12 and encloses a preset number of micropillars 12.

The function of the cell-confining structure in this embodiment is for isolated detection of single cells, overlapping detection of two single cells, or isolated detection of multicellular aggregates, i.e., to avoid contact or adhesion between cells and cells, or between multicellular aggregates and multicellular aggregates during detection, and to confine the size, shape, and morphology of cells, thereby facilitating high-throughput testing. According to different needs, the number of confining surfaces 15 in the cell-confining structure or the shape they enclose can be different. For example, the confining surface 15 included in the cell-confining structure can be a cylindrical surface, or it can be 3 planes that are connected end-to-end to form a triangular cross-sectional shape and enclose a certain number of micropillars, 4 planes that are perpendicular to each other and connected end-to-end to form a rectangular shape and enclose a certain number of micropillars, N planes that are connected end-to-end to enclose an N-sided polygon, or a curved surface with a circular-like cross-section, etc. That is, the cross-sectional shape formed by the confining surfaces 15 is a controllable closed shape, and its area (or can be understood as the number of micropillars it can accommodate in its space) is also controllable. In actual embodiments, depending on the manufacturing process, the cell-confining structure can be a structure integrally formed with the base 11, or a structure bonded to the base 11.

## Embodiment 4

Please refer to the multimodal biophysical characterization apparatus shown in Figure 18. On the basis of the multimodal biophysical characterization apparatus of Embodiments 1-3, at least one of the base 11, micropillars 12, and confining surfaces 15 is provided with a substance 16 that interacts with cells. The substance 16 that interacts with cells is selected from a group consisted of a substance that promotes cell adhesion, a substance that interacts with cell surface receptors, a biologically active substance, and a biologically inactivated substance. Therefore, for the multimodal biophysical characterization apparatus in this embodiment, since at least one of the base 11, micropillars 12, and confining surfaces 15 is coated with a substance that interacts with cells (promotes adhesion), the cell to be characterized is easily affected by its adhesion effect and will enclose part or all of the micropillars.

## Embodiment 5

Please refer to Figure 19. On the basis of the multimodal biophysical characterization apparatus of Embodiment 4, at least one of the base 11, micropillars 12, and confining surfaces 15 is provided with a substance with anti-cell adhesion properties. Therefore, in this embodiment, the substance that interacts with cells (promotes adhesion) 16 and the substance with anti-cell adhesion properties 17 can be purposefully provided on the base 11, micropillars 12, and confining surfaces 15 according to the actual needs of the measurement, so as to further define the cell adhesion range, ensure it falls within the area and shape to be tested, and better regulate the migration and differentiation of cells. Under the condition of controlling the size, shape, and tropic characteristics of the cells, it is possible to controllably induce the cell or multicellular aggregate to be characterized to stay on the micropillars without being pierced to measure cell mechanical force, or to induce specific parts of the cell or multicellular aggregate to be characterized to be pierced by the micropillars to measure cell hardness.

## Embodiment 6

On the basis of the multimodal biophysical characterization apparatus of Embodiment 5, the substance that interacts with cells is provided on the top end of the micropillars; multiple micropillars provided with the substance that interacts with cells form a first preset pattern; and/or the substance with anti-cell adhesion properties is provided on the top end of the micropillars, and multiple micropillars provided with the substance with anti-cell adhesion properties form a second preset pattern. Specifically, micrometer-scale printing technology can be used to print specific patterns of substances with cell adhesion mechanisms, such as (1) extracellular matrix molecules, including but not limited to collagen, fibronectin, vitronectin, laminin, or proelastin; (2) mimics of the extracellular matrix, including but not limited to polypeptides containing the RGD adhesion sequence; (3) substances with a cell adhesion-promoting mechanism, including but not limited to polylysine; substances that interact with cell surface receptors include but are not limited to antibodies, to promote cell attachment in these areas. The so-called preset pattern can be shapes such as a triangle, a quadrilateral, a polygon, a circle, an ellipse, etc. The functions of the preset pattern include: first, controlling cell and cell-cell contact through these patterns formed by substances with cell adhesion mechanisms, to facilitate the demand for high-throughput data acquisition. Second, achieving a dimensionality reduction effect in data processing by making the cell shape uniform, thereby reducing the difficulty of analysis. Third, achieving the purpose of controlling the size, shape, tropism, differentiation state, etc., of cells by providing substances with anti-cell adhesion properties such as BSA or F127 to limit the cell attachment area. It is even possible to regulate the mechanical state of the cell by controlling actin filaments, thereby meeting the requirements of certain special technical scenarios. In actual operation, the patterns of the first preset pattern and the second preset pattern can be the same or different.

### Embodiment 7

Please refer to Figures 20, 21, 22, and 23. The difference from the multimodal biophysical characterization apparatus provided in Embodiment 1 is that a fluorescent substance 18 is provided on the extension part of the micropillar from the top end to the bottom end. It can be on the side surface near the top end of the micropillar, or within the pillar body enclosed by the side surface near the top end of the micropillar, or on the side surface near the bottom end of the micropillar, or within the pillar body enclosed by the side surface near the bottom end of the micropillar, or at any position on the side surface and within the pillar body enclosed by the side surface of the micropillar. The presence of the fluorescent substance can achieve the purpose of more intuitively calibrating the cell or multicellular aggregate to amplify the signal and improve the signal-to-noise ratio. At the same time, the cell or multicellular aggregate to be characterized can be stained with a fluorescent substance of another color, and then the hardness of the cell or multicellular aggregate can be judged by the depth of piercing.

In Embodiments 1-7 of the present application, the material of the micropillar 12 is polydimethylsiloxane (PDMS). In some other main embodiments of the present application, the material of the micropillar 12 can also be other polymer materials, for example, silicon-based polymers, photoresist polymer materials, conductive polymer materials, temperature-sensitive polymer materials, etc.

### Embodiment 8

Please refer to Figure 24. Two multimodal biophysical characterization apparatuses provided by the present application are arranged opposite each other to form a double-sided structure. The outside is the base, and the inside encloses a three-dimensional accommodation cavity for a cell or multicellular aggregate. The volume or height of the three-dimensional accommodation cavity can be regulated according to actual needs. For example, when the cell to be characterized is a single cell on opposite sides, the height of the three-dimensional accommodation cavity can be controlled between 5 nm and 2 mm. When the cell to be characterized is a multicellular aggregate or living tissue, the height and volume can be adjusted to be correspondingly larger, with the height even reaching 5 cm and the volume reaching 30 cm$^3$.

This embodiment provides an apparatus and method for simultaneously measuring the two-sided physical properties of a cell.

S1: Prepare a micropillar chip, with a metal reflective coating on top of its micropillars, and covered with extracellular matrix. The side surfaces of the micropillars and the space between the micropillars are coated with an anti-reflection coating and an anti-cell adhesion coating.

S2: Culture the lung cancer cell line A549 on this chip until fully attached.

S3: Prepare another chip, with only the top ends of the micropillars coated with extracellular matrix protein, and the rest coated with anti-cell adhesion F127, to facilitate the measurement of cell mechanical force.

S4: Place the chip from step S3 inverted on top of the chip containing cancer cells, so that the confining structures are aligned and the two types of cells are in contact.

S5: Equip the top and bottom of the apparatus with a light source (light signal emitting device) and a reflected light receiver (light signal detecting device), respectively, to simultaneously detect the reflected signals from the upper and lower chips, to characterize cell mechanical force and softness/hardness.

S6: Monitor the changes in the physical properties of both types of cells in real-time to establish a corresponding relationship between the trends of change in cell mechanical force and softness/hardness.

Figure 29 shows an example of measuring a multicellular aggregate using a double-sided structure composed of the multimodal biophysical characterization apparatus provided by the present application.

In other different embodiments, two or more multimodal biophysical characterization apparatuses can be formed into a multi-sided structure (Figure 25) to simultaneously measure the magnitude and spatial position distribution of the cell mechanical force and cell hardness of multiple sides of the cell to be characterized or of different cells in a multicellular aggregate.

The purpose of another embodiment is to culture cells in a three-dimensional attachment environment of a multi-sided structure, and to flexibly measure the cell mechanical force and/or softness/hardness of each side.

Experimental steps:

S1. Prepare a base with a micropillar structure. The top of the micropillars is coated with a metal reflective coating, and the side surface of the micropillars is coated with an anti-reflection coating.

S2. Place two bases with micropillar structures on the left and right sides of the base from step S1, so that a trough-like structure is formed inside. The micropillars are coated with a cell adhesion-promoting extracellular matrix protein only on the top, with the rest coated with anti-cell adhesion F127, so that cells only contact the top end of the micropillars, to facilitate the measurement of cell mechanical force.

S3. Coat the periphery of the micropillars at the bottom of the trough-like structure and the space between the micropillars with extracellular matrix, and stretch the base to adjust the spacing between the micropillars, to facilitate the piercing of the cells by the micropillars to characterize the softness/hardness of the cells.

S4. Distribute a cell suspension evenly within the trough-like structure, so that the cells adhere to the multiple surfaces of the micropillars.

S5. Place the apparatus in a cell culture incubator to provide a suitable growth environment for the cells, and culture for a certain period of time (e.g., 48 hours) to allow the cells to fully adhere to the multiple surfaces of the trough-like structure.

S6. During the experiment, an appropriate magnetic field can be applied to the bottom micropillars from outside the apparatus to cause the micropillars at the bottom of the trough-like structure to deform. Use a light source to illuminate the micropillars at the bottom of the trough-like structure and collect the reflected light signal. By analyzing the changes in the intensity and frequency of the reflected light signal, the softness/hardness of the cells on the bottom surface can be observed.

S7. Use a light source to illuminate the micropillars on the side surface of the trough-like structure and collect the reflected light signal. By analyzing the attenuation and dynamic changes of the reflected light signal, the mechanical force of the cells on the side surface can be observed.

S8. Analyze the experimental data to summarize the physical properties of the cells in a three-dimensional attachment environment and their relationship with biological properties such as cell growth and migration.

In some more preferred embodiments, the cell-confining structures are adapted to each other (Figure 26), i.e., two opposing cell-confining structures form an accommodation cavity for the target cell to be characterized.

**Embodiment 9**

The difference from the multimodal biophysical characterization system provided in Embodiment 1 of the present application is that a light signal analysis device can also be connected, used to analyze the light signal to improve the timeliness and operability of processing.

**Embodiment 10**

The multimodal biophysical characterization system provided in this embodiment, in addition to being connected to a light signal analysis device, is also connected to a data processing device. The data processing device is used to compute and process the light signal to obtain the results and spatial distribution information of cell mechanical force and/or cell softness/hardness. This way, complex calculations by hand are not needed, improving the accuracy of data processing and measurement efficiency. At the same time, based on the results and spatial distribution of cell mechanical force and/or cell softness/hardness, predictive information on cell behavior and differentiation direction is given.

**Embodiment 11**

The multimodal biophysical characterization system provided in this embodiment also includes a pressure transmitting device. The pressure transmitting device is used to apply pressure to the cells. The pressure transmitting device can also include pressure sensing and pressure feedback, measuring the value of the specific pressure to regulate the magnitude of the applied force. When no substance with a mechanism for promoting cell adhesion is provided on the micropillars, in order to better achieve the smooth measurement of cell mechanical force and/or cell hardness, external pressure can also be used to cause the cells to move relative to the micropillars in a directional manner. These pressure transmitting devices include, for example, a gravity block, an automated pressure device, a probe, etc.

**Embodiment 12**

The multimodal biophysical characterization system provided in this embodiment also includes a device for causing motion or deformation. The device for causing motion or deformation is used to apply a mechanical force to the multimodal biophysical characterization system. Since the base can be made of a material with a certain degree of flexibility and elasticity, the devices for causing motion or deformation that can be used include but are not limited to a pulling device or an inflation/deflation device. The pulling device is used to apply an appropriate mechanical pulling force to the base to control the stretching motion or deformation of the base in the horizontal direction, and the inflation/deflation device is used to form a motion or deformation on a curved surface of the base.

**Embodiment 13**

The difference in the multimodal biophysical characterization system provided in this embodiment is that the base and/or the micropillars are made of a conductive material, the purpose of which is, in addition to being able to apply electrical stimulation during the process of characterizing the cell or multicellular aggregate, also to achieve the purpose of receiving electrical signals from the biological entity.

**Embodiment 14**

The difference in the multimodal biophysical characterization system provided in this embodiment is that the light signal emitting device has a light source, the light source comprising a first light source arranged at the bottom of the base, and/or a second light source arranged at the side of the base, and/or a third light source located at the top end opposite the bottom and away from the base. The light beam emitted by the first light source and/or the second light source and/or the third light source reaches the micropillars. No special provisions need to be made for the types of the first light source, the second light source, and the third light source arranged at the bottom of the base. This arrangement can, on the whole, improve the measurement throughput, reduce the volume of the system, and enhance the portability of the system. In a preferred embodiment, the second light source can use waveguide illumination. In addition to making the system smaller, the addition of an evanescent wave illumination mode can significantly enhance the signal-to-noise ratio.

**Embodiment 15**

The difference in the multimodal biophysical characterization system provided in this embodiment is that a magnetic field emitting device is added to emit a magnetic field of a predetermined direction and intensity towards the multimodal biophysical characterization apparatus. The magnitude and direction of the magnetic field can be regulated according to actual needs. For example, it can cause all micropillars to tilt in an orderly manner in one direction. And when the cell hardness is relatively high, the magnetic field can restrain the tilting of the micropillars, making the tilting amplitude smaller, and more light reflection signals can be detected. Conversely, when the cell hardness is relatively soft, the tilting amplitude of the micropillars will be larger, and the detectable light reflection signal will become weaker. The cell hardness can be known by using the magnitude of the applied magnetic force and the intensity of the light reflection signal.

**Embodiment 16**

**Measuring nuclear hardness using two independent methods: the magnetic material method and the fluorescence method**

The purpose of this embodiment is to measure nuclear hardness using two independent methods-the magnetic material method and the fluorescence method. The magnetic material method measurement is simpler and does not require an expensive confocal microscope, and its accuracy is cross-validated by the fluorescence method.

S1: Provide a chip whose surface is covered with PDMS nano-magnetic micropillars. The micropillars contain a fluorescent substance and have an extracellular matrix coating.

S2: Culture the macrophage cell line THP-1 on this chip. Contact between the THP-1 cells and the chip is made by controlling the height of the culture medium.

S3: Add lipopolysaccharide (LPS) to the culture medium to activate the THP-1 cells, causing them to transform into M1-type macrophages.

S4: After several days of cultivation, use the magnetic material method to turn on the magnetic field and measure the reflected light attenuation. After turning off the magnetic field, measure the light reflection signal attenuation value and frequency of each micropillar, and sequentially calculate the softness/hardness of the nucleus at each position.

S5: Use paraformaldehyde to fix the cells on the chip, and use DAPI to stain the nuclei.

S6: Using the fluorescence method, use a confocal microscope to perform a Z-axis tomographic scan from the top to the bottom of the micropillars, and analyze the depth of the DAPI signal of the nucleus between the micropillars. When the nuclear hardness is low, the sagging depth of the nucleus between the micropillars is deeper; conversely, when the nuclear hardness is high, the sagging depth of the nucleus is shallower.

S7: This experiment found that after the activation of macrophage THP-1, the sagging depth of the nucleus becomes deeper, indicating that the activation process makes the nuclear hardness softer. The results measured by the magnetic material method and the fluorescence method are the same (Figure 33), proving that the accuracy of measuring nuclear hardness by the magnetic material method has been cross-validated by the fluorescence method.

## Embodiment 17

**This embodiment demonstrates an apparatus and method for measuring the hardness, viscosity, and viscoelasticity at specific locations of a cell**

By combining a magnetic metal and the intensity of the light reflection signal, the measurement of cell hardness is achieved.

The specific operating steps are as follows:

S1: Prepare a micropillar chip, the surface of whose micropillars is covered with a magnetic metal (such as iron, cobalt, nickel, etc.) coating, serving as a reflective layer. Apply an anti-reflection coating to the side surfaces of the micropillars, and at the same time, cover the area above the magnetic metal layer and between the micropillars with extracellular matrix.

S2: Seed cells on the micropillar chip for cultivation. After a cultivation period of one to two days, ensure the cells are fully attached and growing on the chip.

S3: Use a PDMS block of a specific mass on the cells, or apply a certain voltage to a fixed piezoelectric material, thereby applying a downward pressure on the cells, prompting the micropillars to pierce the cells to a certain depth.

S4: Apply a magnetic field to cause all micropillars to tilt in an orderly manner in one direction. Subsequently, turn off the magnetic field and observe the return swing of the deformed micropillars. Depending on the cell's hardness, viscosity, and viscoelasticity, the characteristics of the light reflection signal generated during the swing will change, including dynamic changes, extremum magnitude, and frequency. Cells with higher hardness will limit the tilting amplitude of the micropillars, thereby resulting in a stronger light reflection signal; conversely, cells with lower hardness will cause the tilting amplitude of the micropillars to increase, producing a weaker light reflection signal extremum. The greater the viscosity, the slower the swing. By measuring these light reflection signal characteristics, physical characteristics such as the softness/hardness, viscosity, and viscoelasticity of the cell can be inferred.

## Embodiment 18

**This embodiment provides a device technology for measuring the hardness of a multicellular spheroid, utilizing a magnetic metal and the intensity of the light reflection signal to achieve the measurement of multicellular spheroid hardness. The specific steps are as follows:**

S1: Provide a micropillar chip, with a coating of a magnetic metal (such as iron, cobalt, nickel, etc.) on top of the micropillars, which can serve as a reflective layer; an anti-reflection coating is provided on the side of the micropillars; the area above the magnetic metal layer and between the micropillars is covered with extracellular matrix.

S2: Culture multicellular spheroids on this micropillar chip. After one to two days of cultivation, allow the multicellular spheroids to fully attach and grow on the chip.

S3: Adjust the spacing between the micropillars so that, under the action of gravity, the multicellular spheroids are pierced by the micropillars to a certain depth.

S4: Use a magnetic field to cause all micropillars to tilt in an orderly manner in one direction. When the hardness of the multicellular spheroid is relatively high, it will restrain the tilting of the micropillars, making the tilting amplitude smaller, and more light reflection signals can be detected. Conversely, when the hardness of the multicellular spheroid is relatively soft, the tilting amplitude of the micropillars will be larger, and the detectable light reflection signal will become weaker. Therefore, this device can use the intensity of the light reflection signal to know the hardness of the multicellular spheroid.

**Embodiment 19**

This embodiment introduces a device technology for measuring tissue hardness, achieving the measurement of tissue hardness by utilizing a magnetic metal and the intensity of the light reflection signal. The specific steps are as follows:

S1: Prepare a micropillar chip, the top of whose micropillars is covered with a magnetic metal (such as iron, cobalt, nickel, etc.) coating, which can serve as a reflective layer. Apply an anti-reflection coating to the side surfaces of the micropillars, and cover the area above the magnetic metal layer and between the micropillars with extracellular matrix.

S2: Cut a living tissue into a thin slice with a thickness of about $200\mu m$ using a tissue slicer, and directly lay it on the micropillar chip. Add culture medium and culture for one day to allow the tissue to fully attach to the chip.

S3: Use a PDMS block or a piezoelectric material fixed above the tissue to apply a certain voltage, applying a downward pressure on the tissue, prompting the micropillars to pierce the tissue to a certain depth.

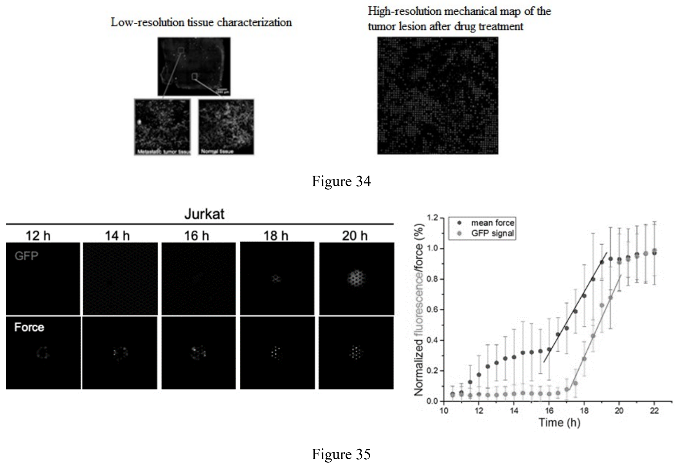

S4: Use a magnetic field to cause all micropillars to tilt in an orderly manner in one direction. When the hardness of the tissue block is relatively high, it will restrain the tilting of the micropillars, leading to a smaller tilting amplitude, thus a stronger light reflection signal can be detected. Conversely, when the hardness of the tissue block is relatively soft, the tilting amplitude of the micropillars will be larger, and the detectable light reflection signal will become weaker. Therefore, this device can use the intensity of the light reflection signal to measure the hardness of the tissue (Figure 34).

**Embodiment 20**

This embodiment provides a method for sorting drug-resistant and non-resistant cells by measuring cell hardness. The specific steps are as follows:

S1: Prepare a micropillar chip, the top of whose micropillars is covered with a magnetic metal (such as iron, cobalt, nickel, etc.) coating, which can serve as a reflective layer. Apply an anti-reflection coating to the side surfaces of the micropillars, and cover the area above the magnetic metal layer and between the micropillars with extracellular matrix.

S2: Culture the lung cancer cell line HCC827 on a double-sided sandwich structure chip composed of micropillar chips. On one side, the upper chip has only the top ends of the micropillars coated with a cell adhesion-promoting layer, with the rest coated with an anti-adhesion layer to prevent cells from growing between the micropillars, for measuring cell mechanical force. On the other side (lower), the top ends, periphery, space between, and base of the micropillars are all coated with a cell adhesion-promoting layer, guiding the micropillars to pierce the cells under the action of the cell adhesion-promoting substance and gravity. After one to two days of cultivation, allow the cells to fully attach and grow on the chip.

S3: Use a pressure transmitting device to apply a certain pressure to the cells, to make the micropillars on the lower chip better pierce the cells. Apply a magnetic field to cause all micropillars to tilt in an orderly manner in one direction, and measure the intensity of the light reflection signal to infer cell hardness. At the same time, measure the cell mechanical force through the reflected light from the upper micropillars.

S4: Record the position coordinates of each cell. Add an EGFR-TKI drug, use a magnetic field to cause all micropillars to tilt in an orderly manner in one direction, and measure the intensity of the light reflection signal to infer cell hardness. Observe the hardness of all cells every half hour. At the same time, monitor the mechanical force of the cells through the light attenuation from the micropillars of the upper chip.

S5: Cell mechanical force is directly related to cell viability. Therefore, the viability and dynamic changes of each cell can be calculated based on the cell mechanical force and its dynamic changes. Thus, under the action of a magnetic field, the magnitude of the tilting amplitude of the micropillars where the cells are located correspondingly leads to the intensity of the light reflection signal. This is used to calculate the softness/hardness and dynamic changes of each cell, and to establish the relationship between cell mechanical force and cell softness/hardness. Among them, a decrease in cell mechanical force means a decrease in cell viability under the action of the drug, i.e., sensitive cells. No significant decrease in cell mechanical force means that cell viability has not changed significantly under the action of the drug, suggesting resistant cells. Thus, the cell hardness characteristics of sensitive and resistant cells are also obtained.

S6: Add a photoactivatable fluorescent dye, and use a UV laser to excite the photoactivatable fluorescent dye, so that the resistant cells of interest carry a red fluorescence.

S7: Use trypsin to remove all cells from the chip, then use a flow cytometer to sort out the cells carrying fluorescence.

S8: Culture the separated cells in a culture medium containing EGFR-TKI, and confirm that the sorted cells have EGFR-TKI resistance. And use the confirmed cell resistance results for supervised or semi-supervised machine learning.

**Embodiment 21**

This embodiment provides a device technology for measuring the mechanical properties of both cells after a cell-cell interaction. The specific steps are as follows:

S1: Prepare a micropillar chip, with a metal reflective coating on top of its micropillars, and covered with extracellular matrix. The side surfaces of the micropillars and the space between the micropillars are coated with an anti-reflection coating and an anti-adhesion coating.

S2: Culture the lung cancer cell line A549 on this chip until fully attached.

S3: Culture the endothelial cell line HUVEC on another chip until fully attached. Alternatively, immune cells, such as T cells or NK cells, can be cultured on this chip to monitor tumor-immune cell interactions. Or, cells can be cultured on a base containing only a confining device but no micropillars, to achieve measurement of only the cells on the other side.

S4: Place the chip from step S3 inverted on top of the chip containing cancer cells, so that the confining structures are aligned and the two types of cells are in contact.

S5: Equip the top and bottom of the apparatus with a light source and a reflected light receiver, respectively, to simultaneously detect the reflected signals from the upper and lower chips, to determine the magnitude of the cell mechanical properties. S6: Monitor the changes in the mechanical properties of both types of cells in real-time to characterize the cell-cell interaction.

**Embodiment 22**

This embodiment provides an apparatus and method for stretching and/or mechanically stimulating cells by causing deformation or mechanical motion of the device by stretching the base, and simultaneously measuring the mechanical force and/or softness/hardness of the cells using reflected light.

S1: Prepare a base with a micropillar structure. The top of the micropillars is coated with a metal reflective coating, and the side surface of the micropillars is coated with an anti-reflection coating.

S2: Coat the top of the micropillars with extracellular matrix protein to promote cell attachment to the top end of the micropillars.

S3: Distribute a cell suspension evenly on the base, so that the cells attach to the top of the micropillars.

S4: Place the apparatus in a cell culture incubator to provide a suitable growth environment for the cells, and culture for a certain period of time (e.g., 24-48 hours) to allow the cells to fully attach to the micropillars.

S5: Use a precise motorized arm or similar device to perform a stretching operation on the base, causing the micropillars to deform or undergo mechanical motion, thereby achieving stretching and/or mechanical stimulation of the cells. In the experiment, the stretching amplitude and speed can be adjusted as needed to simulate different degrees of mechanical stimulation. Or, perform inflation/deflation operations on the bottom of a deformable base to change the curvature of the cell attachment, and to stretch and apply motion stimulation to the cells.

S6: While performing stretching and mechanical stimulation, use a light source to illuminate the micropillars and collect the reflected light signal. By analyzing the intensity and dynamic changes of the reflected light signal, the mechanical force and/or softness/hardness of the cells during the stretching and mechanical stimulation process can be measured in real-time.

S7: Analyze the experimental data to study the changes in the physical properties of the cells under different stretching and mechanical stimulation conditions, and their relationship with biological properties such as cell growth, migration, and differentiation.

**Embodiment 23**

**This embodiment provides an apparatus and method for characterizing the physical properties (cell mechanical force and softness/hardness) of cells in their initial state, relaxed state after pre-stretching, and stretched state. The specific steps are as follows:**

S1: Prepare three bases with a micropillar structure. The top of the micropillars is coated with a metal reflective coating, and the side surface of the micropillars is coated with an anti-reflection coating. Coat the top of the micropillars with extracellular matrix protein to promote cell attachment to the top end of the micropillars.

S2: Pre-stretch one base to the desired extent, then distribute a cell suspension evenly on the pre-stretched base. After the cells have fully attached to the top of the micropillars, release the base to allow it to contract, thereby putting the cells in a relaxed state.

S3: Distribute a cell suspension evenly on another base, so that the cells attach to the top of the micropillars. After the cells have fully attached, stretch the base to put the cells in a stretched state.

S4: Distribute a cell suspension evenly on a third base, without any treatment.

S5: Place the three apparatuses separately in a cell culture incubator to provide a suitable growth environment for the cells. For the three different stretched states of the cells (S1, S2, S3), use a light source to illuminate the micropillars and collect the reflected light signal, respectively. By analyzing the intensity and dynamic changes of the reflected light signal, the mechanical force of the cells in different states can be measured in real-time.

S6: Use a pressure transmitting device to pierce the cells with the micropillars, add a magnetic field, and characterize the softness/hardness of the cells by detecting the light reflection signal.

S7: Analyze the experimental data to compare the changes in the physical properties of the cells in their initial state, relaxed state after pre-stretching, and stretched state, and the corresponding relationship between cell hardness and cell mechanical force.

**Embodiment 24**

**This embodiment provides a method for changing the equivalent hardness of micropillars with a magnetic field parallel to the micropillars, to provide dynamic mechanical stimulation (stiffness) to the cells, and to optimize the measurement range of the micropillars, so that the force to be measured falls within the measurement range of the micropillar sensor. The specific steps are as follows:**

S1. Prepare a base with a micropillar structure. The top of the micropillars is coated with a metal reflective coating, and the side surface of the micropillars is coated with an anti-reflection coating.

S2. Coat the top of the micropillars with extracellular matrix protein to promote cell attachment to the top end of the micropillars.

S3. Distribute a cell suspension evenly on the base, so that the cells attach to the top of the micropillars.

S4. Place the apparatus in a cell culture incubator to provide a suitable growth environment for the cells, and culture for a certain period of time (e.g., 24-48 hours) to allow the cells to fully attach to the micropillars.

S5. During the experiment, by applying a magnetic field parallel to the micropillars, a pulling force is applied upwards on the micropillars, making it more difficult for the micropillars to deform laterally, which is equivalent to increasing the hardness of the micropillars. By adjusting the intensity of the magnetic field, dynamic stiffness stimulation can be applied to the cells, while also optimizing the measurement range of the micropillars, so that the force to be measured falls within the measurement range of the micropillar sensor.

S6. While performing dynamic stiffness stimulation, use a light source to illuminate the micropillars and collect the reflected light signal. By analyzing the intensity and dynamic changes of the reflected light signal, the mechanical force and/or softness/hardness of the cells under different stiffness stimuli can be measured in real-time.

S7. Analyze the experimental data to study the changes in the physical properties of the cells under different stiffness stimuli, and their relationship with biological properties such as cell growth, migration, and differentiation.

**Embodiment 25**

**A method for measuring the interaction between immune cells and molecules and the cell activation process, by characterizing cell mechanical force and hardness** This embodiment provides a method for evaluating the interaction between immune cells (such as T cells) and molecules (such as CD3 antibody) and the cell activation process by measuring the changes in the mechanical force and hardness of the immune cells. The expression of green fluorescent protein is used as a marker for cell activation and is cross-validated with the measurement results of cell mechanical force and hardness.

S1. Prepare a mechanics chip, the top ends of whose micropillars are covered with CD3 antibody; the side surfaces of the micropillars and the space between the micropillars are treated with Pluronic F127 for anti-adhesion.

S2. Add the NFAT reporter (eGFP) Jurkat recombinant cell line to the chip. The CD3 antibody will attract the T cells to contact the chip, and this T cell line will express green fluorescent protein when activated by the CD3 antibody.

S3. Use a multimodal biophysical characterization system to monitor the changes in the mechanical properties of the cells and the expression of green fluorescent protein. By analyzing the changes in the mechanical force of the cells at different time points, the interaction between immune cells and molecules and the cell activation process can be studied.

S4. Use a pressure transmitting device to pierce the cells with the micropillars, add a magnetic field, and characterize the hardness of the cells by detecting the light reflection signal.

S5. Analyze the experimental data to monitor the changes in the mechanical force and hardness of the cells during the interaction between immune cells and molecules and the cell activation process. At the same time, observe the expression of green fluorescent protein and cross-validate with the measurement results of cell mechanical force and hardness to evaluate the activity of the cells under different activation conditions.

The experiment found that cell mechanical force detects cell activation earlier and more sensitively, and in the process of dynamic changes in cell mechanical force, it was found that mechanical force is activated around the cells before cell activation. Specific features can be used to predict the state of the cells and the time of activation (Figure 35).

**Embodiment 26**

**A method for distinguishing activated T cells from unactivated T cells by measuring cell mechanical force and hardness, and for knowing their activation ratio.** This embodiment provides a method for distinguishing activated T cells from unactivated T cells by measuring cell mechanical force and hardness, and for knowing their activation ratio. This method can be used for the characterization of cell therapy samples and for predicting the success probability of cell therapy.

S1. Culture the T cell line NFAT reporter (eGFP) Jurkat cells on a mechanics chip. Control the amount of culture medium to bring the T cells into contact with the multimodal biophysical characterization apparatus, and measure the magnitude of the mechanical force of the unactivated cells.

S2. Add a medium containing CD3 antibody, CD28 antibody, and cytokines to the culture medium to activate the T cells. When the T cells are activated by the antibodies, they will express green fluorescent protein.

S3. Monitor the changes in the mechanical force and the expression of green fluorescent protein of each T cell in real-time.

S4. Use a pressure transmitting device to pierce the cells with the micropillars, add a magnetic field, and characterize the hardness of the cells by detecting the light reflection signal.

S5. It was found that when T cells are activated, the cell mechanical force will gradually increase, which has a positive correlation with the green fluorescence.

S6. Input the changes in cell mechanical force and hardness of each T cell during the activation process into a database. Use a machine learning method to establish a model that can use the features of cell mechanical force and hardness, spatial distribution, and temporal dynamic changes to judge the degree of activation of T cells.

S7. From the peripheral blood mononuclear cells (PBMCs) of a donor, use CD4 and CD8 antibodies to isolate T cells.

S8. Culture the isolated T cell pool on a mechanics chip. Control the amount of culture medium to bring all T cells into contact with the mechanics chip.

S9. Record the magnitude distribution of the mechanical force of all T cells. Use the previously established model for computational analysis to know the ratio of activated and non-activated T cells in the donor's body. This value can be used to predict the success probability of cell therapy.

**Embodiment 27**

**A technology for applying microfluidics and a mechanics chip to the in vitro culture monitoring of CAR-T cell therapy**

This embodiment provides a technology for applying microfluidics and a mechanics chip to the in vitro culture monitoring of CAR-T cell therapy, in order to more effectively monitor and evaluate the growth, activation, and efficacy of CAR-T cells in an in vitro environment.

S1. The top ends of the micropillars of the multimodal biophysical characterization apparatus chip are covered with CD3 and CD28 antibodies; the side surfaces of the micropillars and the space between the micropillars are treated with Pluronic F127 for anti-adhesion.

S2. Lay the chip on the bottom of a microfluidic device.

S3. Inject T cells isolated from the peripheral blood mononuclear cells (PBMCs) of a donor into the microfluidic device, and activate the T cells. At the same time, judge whether all T cells have been fully activated by monitoring the cell mechanical force.

S4. After the T cells are fully activated, add lentivirus for transduction, so that the T cells express a chimeric antigen receptor with an antibody-specific sequence (scFv) that can recognize a cancer cell surface protein.

S5. Take out the transduced T cells (CAR-T) from the microfluidic device and continue to culture and expand them.

S6. Lay a PDMS microwell film on a mechanics chip, and culture tumor cells, tumor multicellular spheroids, or tumor organoids on a mechanics chip containing microwells. The cells will grow in an orderly manner in the microwells.

S7. Add CAR-T cells to the mechanics chip containing tumor cells, and monitor the cell mechanical force of the tumor cells in real-time. When the CAR-T cells can effectively kill the tumor cells, the cell mechanical force of the tumor cells will drop sharply.

Through this embodiment, we can more effectively monitor and evaluate the mechanical properties of CAR-T cells during the process of activation, expansion, and killing of tumor cells in an in vitro environment, thereby providing more accurate data support for CAR-T cell therapy.

**Embodiment 28**

**A technology for applying microfluidics and a mechanics chip to the in vitro culture monitoring of CAR-T cell therapy.**

This embodiment provides a technology for applying microfluidics and a mechanics chip to the in vitro culture monitoring of CAR-T cell therapy, in order to more effectively monitor and evaluate the growth, activation, and efficacy of CAR-T cells in an in vitro environment.

S1. The top ends of the micropillars of the multimodal biophysical characterization apparatus are covered with CD3 and CD28 antibodies; the side surfaces of the micropillars and the space between the micropillars are treated with Pluronic F127 for anti-adhesion.

S2. Lay the multimodal biophysical characterization apparatus on the bottom of a microfluidic device.

S3. Inject T cells isolated from the peripheral blood mononuclear cells (PBMCs) of a donor into the microfluidic device, and activate the T cells. At the same time, judge whether all T cells have been fully activated by monitoring the cell mechanical force.

S4. After the T cells are fully activated, use CRISPR-Cas9 technology to modify the T cell receptors (TCR) of the T cells so that they can express the ability to recognize cancer cell antigens.

S5. Take out the modified TCR-T cells from the microfluidic device and continue to culture and expand them.

S6. Lay a PDMS microwell film on a mechanics chip, and culture tumor cells, tumor multicellular spheroids, or tumor organoids on a mechanics chip containing microwells. The cells will grow in an orderly manner in the microwells.

S7. Add TCR-T cells to the mechanics chip containing tumor cells, and monitor the cell mechanical force of the tumor cells in real-time. When the TCR-T cells can effectively kill the tumor cells, the cell mechanical force of the tumor cells will drop sharply.

Through this embodiment, we can more effectively monitor and evaluate the mechanical properties of TCR-T cells during the process of activation, expansion, and killing of tumor cells in an in vitro environment, thereby providing more accurate data support for TCR-T cell therapy.

**Embodiment 29**

**Using a multimodal biophysical characterization apparatus to monitor changes in cell mechanical force to determine the effectiveness of an antibody-drug conjugate in treating suspension cancer**

This embodiment provides a method for using a multimodal biophysical characterization apparatus to monitor changes in cell mechanical force to determine the effectiveness of an antibody-drug conjugate in treating suspension cancer.

S1. Cover the top ends of the micropillars of the multimodal biophysical characterization apparatus with the antibody-drug conjugate. The side surfaces of the micropillars and the space between the micropillars are treated with Pluronic F127 for anti-adhesion.

S2. Culture blood cancer cells on this apparatus. The antibody coating can attract the blood cancer cells to the apparatus and bring them into contact with it.

S3. Monitor the cell mechanical force of the blood cancer cells in real-time by detecting the intensity of the light reflection signal. When the antibody-drug conjugate can effectively inhibit cell growth or cause the cells to enter apoptosis, the cell mechanical force will decrease sharply.

By using a multimodal biophysical characterization apparatus to monitor the changes in cell mechanical force, we can better understand the therapeutic effect of the antibody-drug conjugate on suspension cancer. Real-time monitoring of cell mechanical force helps to better evaluate the efficacy of the antibody-drug conjugate, thereby optimizing drug design and treatment plans.

**Embodiment 30**

**Using a multimodal biophysical characterization apparatus to monitor changes in cell mechanical force and hardness to determine the effectiveness of an antibody-drug conjugate in treating adherent cancer.** This embodiment provides a method for evaluating the therapeutic effect of an antibody-drug conjugate on adherent cancer by monitoring the changes in cell mechanical force and hardness.

S1. Cover the top ends of the micropillars of the multimodal biophysical characterization apparatus with extracellular matrix. The side surfaces of the micropillars, the space between the micropillars, and the base are treated with Pluronic F127 for anti-adhesion.

S2. Culture lung cancer cells on the multimodal biophysical characterization apparatus. After one day of cultivation, allow the cells to fully attach to the apparatus.

S3. Add the antibody-drug conjugate to the culture medium.

S4. Monitor the cell mechanical force and hardness of the lung cancer cells in real-time by detecting the intensity of the light reflection signal. When the antibody-drug conjugate effectively inhibits cell growth or causes the cells to enter apoptosis, the cell mechanical force and hardness will decrease significantly.

By monitoring the changes in cell mechanical force and hardness with a multimodal biophysical characterization apparatus, important evidence can be provided for evaluating the therapeutic effect of an antibody-drug conjugate on adherent cancer.

**Embodiment 31**

**Using the monitoring of changes in the cell mechanical force of living tissue to determine the therapeutic effect of an antibody-drug conjugate**

This embodiment aims to provide a method for evaluating the therapeutic effect of an antibody-drug conjugate on tumor cells by monitoring the changes in the cell mechanical force of living tissue.

S1. The top ends of the mechanics chip micropillars are covered with extracellular matrix. The side surfaces of the micropillars and the space between the micropillars are treated with Pluronic F127 for anti-adhesion.

S2. Use a tissue slicer to cut a fresh tumor tissue into a thin slice with a thickness of 200 micrometers.

S3. Lay the living tissue slice on the mechanics chip. After one day of cultivation, allow the tissue to fully attach to the chip. It is known that the cell mechanical force of a tumor cell block is stronger than that of a non-tumor block.

S4. Add the antibody-drug conjugate to the culture medium.

S5. Monitor the cell mechanical force of the tumor cell block and the non-tumor cell block in real-time by detecting the intensity of the light reflection signal. When the antibody-drug conjugate effectively inhibits cell growth or causes the cells to enter apoptosis, the cell mechanical force will decrease significantly.

S6. In addition, by comparing the changes in the cell mechanical force of the tumor cell block and the non-tumor cell block, it is possible to know whether the antibody-drug conjugate can specifically kill the tumor cells, and to evaluate the effect on normal cells.

This embodiment, by monitoring the changes in the cell mechanical force of living tissue, provides important evidence for evaluating the therapeutic effect of an antibody-drug conjugate on tumor cells.

**Embodiment 32**

**A preparation method for a multimodal biophysical characterization apparatus**

This embodiment provides a preparation method for a multimodal biophysical characterization apparatus, which achieves the reflection of an optical signal by coating a metal reflective layer on the top of micro-nano pillars.

S1. Spin-coat a layer of photoresist on the micro-nano pillar array. This step can protect the side surfaces of the micro-nano pillars and avoid metal deposition during subsequent processing.

S2. Use oxygen plasma etching technology to expose the tops of the micro-nano pillars. This step ensures that the metal coating only covers the tops of the micro-nano pillars and does not affect the side surfaces.

S3. Deposit a metal coating using an electron beam evaporator. This step will form a metal reflective layer on the exposed tops of the micro-nano pillars, achieving the reflection of an optical signal. If a magnetic metal is used, a magnetic metal reflective layer can be formed on the tops of the micro-nano pillars, achieving both the reflection of an optical signal and a response to a magnetic field. This can also be repeated multiple times to achieve the deposition of different metals or coatings with different functions (Figure 30).

S4. Remove the photoresist. This step can clear the residual photoresist, ensuring that the metal layer only covers the tops of the nano-pillars. And use a scanning electron microscope to characterize the prepared chip samples and confirm the successful setting of the metal coating (Figure 30).

Through the above preparation process, we can obtain a micropillar chip with a metal coating or a magnetic metal top, used for the reflection and detection of an optical signal, as well as for achieving a response to a magnetic field.

**Embodiment 33**

**A conversion method between micropillar deformation and the force applied**

This embodiment provides a conversion method between deformation and the force applied, based on the deformation of the micropillar and Hooke's law formula, and considers the effect of the deformation at the bottom of the micropillar.

S1. Calculate the ideal spring constant of the micropillar, k_bend:

$$k\_bend = (3/64) * \pi * E * D^4 / L^3$$

where E is Young's modulus, D is the diameter of the micropillar, and L is the length of the micropillar.

S2. Calculate the tilting coefficient, T_tilt(v):

$$T\_tilt(v) = a * (1 + v) / (2 * \pi) * \{2 * (1 - v) + (1 - 1 / (4 * (1 - v)))\}$$

where v is Poisson's ratio, and a is a correction coefficient.

S3. Calculate the deformation proportionality factor, a:

$$a = \delta\_bend / (\delta\_bend + \delta\_shear + \delta\_tilt)$$

where $\delta$_bend is the bending deformation of the micropillar, $\delta$_shear is the shear deformation, and $\delta$_tilt is the tilting deformation at the bottom of the micropillar.

S4. Calculate the corrected spring constant, k_corr:

$$k\_corr = k\_bend \times a$$

S5. Calculate the force on the micropillar, F:

$$F = k\_corr \times \delta$$

where $\delta$ is the deformation of the micropillar.

S6. Calculate the deformation at the top of the micropillar,

$$\delta\_tilt \text{ (Figure 30): } \delta\_tilt = 8 * T\_tilt(v) * (L/D)^2 * (4/\pi) * F / (E*D)$$

Through the above calculation method, we can consider the effect of the deformation at the bottom of the micropillar and calculate the force on the micropillar more accurately.

## Embodiment 34

**A method for calibrating a multimodal biophysical characterization system using a flow field**

This embodiment aims to provide a method for calibrating a multimodal biophysical characterization system using a flow field.

S1. The top of the PDMS micropillars has a metal reflective coating, and the space between and the side surfaces of the micropillars have an anti-reflection coating.

S2. Lay this micropillar chip on the bottom of a microfluidic channel (Figure 32).

S3. Inject fluid at different flow rates into the microchannel inlet. Illuminate the chip from below the apparatus with a laser beam or visible light, and at the same time, measure the light reflection signal generated at the top of the micropillars from below the apparatus. When the flow rate is faster, the bending amplitude of the micropillars becomes larger, and the light reflection signal becomes weaker. It has been experimentally confirmed that, within a certain range of deformation, the flow rate and the light reflection signal show a linear relationship (Figure 31). Therefore, the state of the fluid can be inferred from the intensity of the light reflection signal generated by the micropillar deformation.

This embodiment provides a method for measuring a flow field and performing flow rate calibration using the reflected light from a micropillar sensor. Within a certain range of deformation, the deformation and the attenuation of the light reflection signal are linearly related. The state of the fluid can be inferred from the intensity of the light reflection signal generated by the micropillar deformation.

## Embodiment 35

**Using a multimodal biophysical characterization system to characterize immune-tumor cell interactions and evaluate the effect of drug intervention**

This embodiment aims to provide the relationship between the physical properties of tumor cells and their interaction with immune cells, as well as the effect of drug intervention on this relationship.

S1. Prepare the chip: Select a cell mechanics chip with a micropillar structure. The top of the micropillars is coated with a suitable extracellular matrix (ECM) to promote the attachment and growth of tumor cells and immune cells. Use an anti-adhesion treatment on the side surfaces of the micropillars and in the space between them.

S2. Seed tumor cells and immune cells: Seed tumor cells and immune cells (such as NK cells or T cells) separately on the mechanics chip, allowing them to interact.

S3. Monitor the mechanical properties of the tumor cells: Use the light reflection signal and micropillar deflection to monitor in real-time the changes in the cell mechanical force and hardness of the tumor cells during their interaction with the immune cells.

S4. Observe the killing effect of immune cells on tumor cells: It was found that the magnitude of the mechanical properties of tumor cells is positively correlated with their hardness. Softer tumor cells (with smaller cell mechanical force) are difficult for immune cells to recognize and kill.

S5. Drug intervention: Add the drug JAS, which makes tumor cells harder, to the culture medium, and observe the changes in the cell mechanical force and hardness of the tumor cells.

S6. Evaluate the effect of drug intervention: Under the action of the drug, the tumor cells become harder, and the cell mechanical force increases (Figure 32). It was observed that the killing effect of immune cells on tumor cells was enhanced accordingly.

Summary: This embodiment demonstrates how to use a cell mechanics chip to characterize the interaction between immune and tumor cells, as well as the effect of drug intervention on this relationship. By studying the mechanical properties of tumor cells, a basis can be provided for the optimization of immunotherapy strategies.

## Embodiment 36

**A multimodal lung organ chip and method of use**

This embodiment aims to provide a multimodal lung organ chip and method of use. Normal and tumor lung extracted cells are seeded on a double-sided sandwich structure system. The chip base is a flexible material that can deform through inflation/deflation to simulate the breathing motion of the lungs. During the process of adding chemotherapy drug stimulation, the light reflection signal and magnetic field-induced micropillar deflection are used to monitor the changes in cell mechanical force and hardness, to measure the killing effect on tumors and the effect on normal cells, in order to evaluate the targeting of the drug.

S1. Prepare a double-sided structure system. The chip base is a flexible material. The top part of the micropillars is coated with extracellular matrix (ECM). The side surfaces of the micropillars and the space between them are treated with an anti-adhesion treatment, for example, Pluronic F127.

S2. Seed normal lung cells on one side of the chip and lung tumor cells on the other side, allowing the cells to fully attach to the chip.

S3. Place the double-sided chip in a device that can deform through inflation/deflation to simulate the breathing motion of the lungs. In addition, the curved base also simulates the alveolar space in the body, providing a curved adhesion microenvironment for the cells or tissue.

S4. Add chemotherapy drugs to stimulate the normal lung cells and lung tumor cells. During the stimulation process, use the light reflection signal to monitor the interaction between the cells and the micropillars, thereby detecting the changes in cell mechanical force in real-time. Use a magnetic field to induce micropillar deflection, measure the force exerted by the cells on the micropillars, and further evaluate the cell hardness and its dynamic changes.

S5. Analyze the light reflection signal and magnetic field-induced micropillar deflection data, compare the effects of the chemotherapy drug on normal lung cells and lung tumor cells, and measure the killing effect on tumors and the effect on normal cells. Evaluate the targeting of the chemotherapy drug based on the experimental results to provide a basis for drug screening and research.

**Embodiment 37**

**Physical spatial omics monitoring of living tissue slices to distinguish between tumor and normal tissue and to evaluate the effect of drug treatment**

This embodiment is a multimodal tissue physical characterization chip and method of use. By monitoring the spatial omics features of the mechanical properties of living tissue slices, tumor and normal tissue regions are distinguished, and the effect of drug treatment is evaluated.

S1. Prepare living tissue slices: Obtain a living tissue sample (containing tumor and normal tissue) and prepare it into thin slices.

S2. Measure the mechanical properties of the tissue slices: Use an atomic force microscope (AFM) or similar technology to measure the mechanical features and hardness distribution of the tissue slices.

S3. Distinguish between tumor and normal tissue: Measure the mechanical properties of the tissue slices: Monitor the mechanical force through the light reflection signal of the mechanics chip, and measure the hardness by activating the deflection of micropillars pierced into the tissue with a magnetic field, and verify with an atomic force microscope (AFM) or similar technology.

S4. Drug treatment: Add an anti-tumor drug to the living tissue slices and treat for a certain period of time.

S5. Evaluate the effect of drug treatment: After drug treatment, measure the changes in mechanical force and hardness at the tumor location. It was observed that the mechanical force in the tumor area was significantly reduced, with a more uniform visual display (Figure 34), while the hardness did not decrease significantly, indicating that the response speed of mechanical force is faster than that of hardness.

Summary: This embodiment demonstrates how to distinguish between tumor and normal tissue regions, as well as evaluate the effect of drug treatment, by monitoring the mechanical property spatial omics of living tissue slices. This method is expected to provide a basis for early tumor diagnosis and drug efficacy evaluation.

It needs to be explained that, although the above embodiments have been described in this text, this does not thereby limit the scope of patent protection of the present application. Therefore, based on the innovative concepts of the present application, changes and modifications made to the embodiments described in this text, or equivalent structural or equivalent process transformations made using the content of the description and drawings of the present application, and the direct or indirect application of the above technical solutions to other related technical fields, are all included within the scope of patent protection of the present application.

**Claims**

1.  A multimodal biophysical characterization apparatus, **characterized in that**, comprising: a base, and,

    a micropillar or micropillar array having at least one micropillar disposed on the base, which is capable of undergoing a state change under the action of cell mechanical force and/or magnetic force,
    wherein the micropillar comprises a bottom end connected to the base, a side surface, a pillar body enclosed by the side surface, and a top end away from the base and opposite to the bottom end, and wherein a light reflection layer is disposed at any position on the top end, side surface, and/or within the pillar body of the micropillar, and wherein a magnetic material is disposed at any position on the top end, side surface, and/or within the pillar body of the micropillar, and preferably, a magnetic metal light reflection layer is disposed on the top end of said micropillar.

2.  The multimodal biophysical characterization apparatus according to claim 1, **characterized in that**, a light reflection layer is disposed on the top end of the micropillar, and an anti-reflection layer is disposed on the side surface of the micropillar and/or the base surface.

3.  The multimodal biophysical characterization apparatus according to claim 1, **characterized in that**, further comprising a plurality of cell confinement structures disposed on the base, the cell confinement structures comprise one or more confinement surfaces, the confinement surfaces being a plane or a curved surface, the confinement surfaces being perpendicular to the plane where the base is located, and are connected to the base or integrally formed with the base, and the height of the confinement surfaces being higher than the micropillars and enclosing a predetermined number of micropillars therein.

4.  The multimodal biophysical characterization apparatus according to any one of claims 1 to 3, **characterized in that**, at least one of the base, micropillar, and confinement surface is provided with a substance that interacts with cells, the substance that interacts with cells is comprises one or more of a substance that promotes cell adhesion, a substance that is interactable with cell surface receptors, a biologically active substance, and a biologically inactivated substance.

5.  The multimodal biophysical characterization apparatus according to claim 4, **characterized in that**, at least one of the base, micropillar, and confinement surface is provided with a substance having an anti-cell adhesion effect.

6.  The multimodal biophysical characterization apparatus according to claim 5, **characterized in that**, the substance that interacts with cells is disposed on the top end of the micropillar, and a plurality of micropillars provided with the substance that interacts with cells form a first predetermined pattern;
    and/or
    the substance having an anti-cell adhesion effect is disposed on the top end of the micropillar, and a plurality of micropillars provided with the substance having an anti-cell adhesion effect form a second predetermined pattern.

7.  The multimodal biophysical characterization apparatus according to claim 1, **characterized in that**, a fluorescent substance is disposed on an extension part from the top end to the bottom end of the micropillar.

8.  The multimodal biophysical characterization apparatus according to claim 1, **characterized in that**, the base and/or micropillar are made of a conductive material.

9.  A multimodal biophysical characterization system, **characterized in that**, comprising: at least one of multimodal biophysical characterization apparatuses according to any one of claims 1 to 8;

    a light signal emitting device, wherein the light signal emitting device is arranged to emit predetermined light;
    a light signal detecting device, wherein the light signal detecting device is arranged to detect light reflected from the light reflection layer;
    and
    a magnetic field emitting device, wherein the magnetic field emitting device is arranged to generate a magnetic field to produce a magnetic force action with the magnetic material; wherein the light emitted by the light signal emitting device illuminates the light reflection layer through an incident light path,
    and the light reflected by the light reflection layer enters the light signal detecting device through a reflection light path.

10. The multimodal biophysical characterization system according to claim 9, **characterized in that**, further comprises a light signal analysis device, wherein the light signal analysis device is arranged to analyze the light signal.

11. The multimodal biophysical characterization system according to claim 10, **characterized in that**, further comprises a data processing device, wherein the data processing device is arranged to compute and process the light signal to obtain results of cell mechanical force and/or cell softness/hardness and their spatial distribution.

12. The multimodal biophysical characterization system according to claim 9, **characterized in that**, further comprises a pressure application device, wherein the pressure application device is arrange to apply pressure to cells.

13. The multimodal biophysical characterization system according to claim 9, **characterized in that**, the light signal emitting device comprises a light source, the light source comprises a first light source disposed at the bottom of the base and/or a second light source disposed at the side of the base and/or a third light source disposed at the top end away from the base and opposite to the bottom end, and the light emitted by the first light source and/or second light source and/or third light source is arranged to reach the micropillars.

14. The multimodal biophysical characterization system according to claim 9, **characterized in that**, further comprises a device for causing movement or deformation, the device for causing movement or deformation is arranged to cause deformation of the multimodal biophysical characterization system and thereby apply a mechanical force to the multimodal biophysical characterization system, preferably the device for causing movement or deformation comprises a pulling device and/or an inflation-deflation device.

15. The multimodal biophysical characterization system according to claim 9, **characterized in that**, a double-sided structure or a multi-sided structure is constituted by two or more of the multimodal biophysical characterization apparatuses, wherein the outer side of the double-sided structure or multi-sided structure is the base, and the inner side encloses a three-dimensional accommodation cavity for cells.

16. The multimodal biophysical characterization system according to claim 15, **characterized in that**, when a double-sided or multi-sided structure is constituted by two or more of the multimodal biophysical characterization apparatuses, the cell confinement structures are adapted to each other.

17. A method for performing physical characterization of cells using the multimodal biophysical characterization system according to any one of claims 9 to 16, **characterized in that**, comprising the following steps:

emitting predetermined light using the light signal emitting device;
detecting the light after the action of the multimodal biophysical characterization apparatus using the light signal detecting device.

18. The method according to claim 17, **characterized in that**, further comprising a step of emitting a magnetic field of a predetermined direction and intensity using the magnetic field emitting device,

optionally, before, during, or after the physical characterization, further comprising a step of: applying stimuli of different types and intensities to the sample to be characterized;
optionally, the stimuli being at least one of physical stimuli, chemical stimuli, and biological stimuli; optionally, the physical stimuli, chemical stimuli, and biological stimuli comprises one or two or more of drugs, mechanical force, hardness, biochemistry, electric field, flow field, tropic guidance, radiation;
optionally, before, during, or after the physical characterization, further comprises the step of: performing marking, fixing, ablating, cutting, extracting, or sorting on a specific position or region of the sample to be characterized;
optionally, performing a comparative analysis with methods including protein staining, histochemical staining, or single-cell sequencing before, during, or after the physical characterization.

19. The method according to claim 18, **characterized in that**, further comprising a light signal analysis step: using the light signal analysis device to perform a comparative analysis of the reflected light before and after the fluid flows through the multimodal biophysical characterization apparatus, and/or, using the light signal analysis device to perform a comparative analysis of the reflected light before and after the magnetic field emitting device generates a magnetic field of a predetermined direction and intensity, to obtain information on the cell mechanical force and/or cell hardness of the sample to be characterized;

the cell mechanical force information comprising magnitude, direction, frequency, distribution, and dynamic change over time of the cell mechanical force at a specific position of a cell or multicellular aggregate, and the cell hardness information comprising magnitude of hardness and spatial distribution of different layers at a specific position within a cell or multicellular aggregate, and its dynamic change over time.

20. The use of the multimodal biophysical characterization apparatus according to claim 1 for the fields of synthetic biology, cell therapy, drug discovery, characterization of intercellular interactions, early tumor screening, or precision medicine.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

Figure 17

Figure 18

Figure 19

Figure 20

Figure 21

Figure 22

Figure 23

Figure 24

Figure 25

Figure 26

The light source can be positioned below

The light source can be positioned on the side,
a CCD, CMOS, PMT, PIN, or APD can be placed underneath the micropillars as the optical signal detection device

Optical signal detection device

The light source can be positioned above

Figure 27

Figure 28

Figure 29

Figure 30

Figure 31

Figure 32

Figure 33

Figure 34

Figure 35

## INTERNATIONAL SEARCH REPORT

| International application No. |
| --- |
| **PCT/CN2024/083363** |

**A. CLASSIFICATION OF SUBJECT MATTER**

C12M 1/34(2006.01)i; C12M 1/00(2006.01)i; C12Q 1/02(2006.01)i; G06V20/69(2022.01)i

According to International Patent Classification (IPC) or to both national classification and IPC

**B. FIELDS SEARCHED**

Minimum documentation searched (classification system followed by classification symbols)

IPC:C12M,C12Q,G06V

Documentation searched other than minimum documentation to the extent that such documents are included in the fields searched

Electronic data base consulted during the international search (name of data base and, where practicable, search terms used)

CJFD, CNTXT, ENTXT, ENTXTC, PUBMED, SpringerLink, ISI Web of Knowledge, Elsevier Science Direct, Wiley InterScience, Nature, Science, CNKI, 万方, WANFANG: 细胞机械力, 细胞牵引力, 细胞力, 微柱, 反射, 磁性, mechanical force, micro-column, array.

**C. DOCUMENTS CONSIDERED TO BE RELEVANT**

| Category* | Citation of document, with indication, where appropriate, of the relevant passages | Relevant to claim No. |
| --- | --- | --- |
| PX | WO 2023046166 A1 (RUIXIN (FUZHOU) TECHNOLOGY CO., LTD.) 30 March 2023 (2023-03-30) claims 1-21, and description, paragraphs 52 and 219-225 | 1-20 |
| X | US 2015300953 A1 (THE UNIVERSITY OF NORTH CAROLINA AT CHAPEL HILL) 22 October 2015 (2015-10-22) claims 1-83, description, paragraphs 15-63, and figure 1 | 1-20 |
| X | CN 106338500 A (BEIJING INSTITUTE OF NANOENERGY AND NANOSYSTEMS) 18 January 2017 (2017-01-18) claims 1-14 | 1-20 |
| Y | US 2018372635 A1 (THE REGENTS OF THE UNIVERSITY OF CALIFORNIA) 27 December 2018 (2018-12-27) claims 1-51 | 1-20 |
| Y | US 2020116696 A1 (THE REGENTS OF THE UNIVERSITY OF CALIFORNIA) 16 April 2020 (2020-04-16) claims 1-79, and description, paragraphs 115-117 | 1-20 |

☑ Further documents are listed in the continuation of Box C.    ☑ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **21 June 2024** | **03 July 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/083363** |

**C.    DOCUMENTS CONSIDERED TO BE RELEVANT**

| Category* | Citation of document, with indication, where appropriate, of the relevant passages | Relevant to claim No. |
| --- | --- | --- |
| Y | CN 109827928 A (SOUTHEAST UNIVERSITY) 31 May 2019 (2019-05-31)<br>claims 1-7, and embodiment 1 | 1-20 |

Form PCT/ISA/210 (second sheet) (July 2022)

**INTERNATIONAL SEARCH REPORT**
Information on patent family members

International application No.

**PCT/CN2024/083363**

| Patent document cited in search report | | | Publication date (day/month/year) | Patent family member(s) | | | Publication date (day/month/year) |
|---|---|---|---|---|---|---|---|
| WO | 2023046166 | A1 | 30 March 2023 | AU | 2022351105 | A1 | 09 May 2024 |
| US | 2015300953 | A1 | 22 October 2015 | US | 9952149 | B2 | 24 April 2018 |
| | | | | WO | 2014085804 | A1 | 05 June 2014 |
| | | | | EP | 2926115 | A1 | 07 October 2015 |
| | | | | EP | 2926115 | A4 | 06 July 2016 |
| | | | | EP | 2926115 | B1 | 26 May 2021 |
| CN | 106338500 | A | 18 January 2017 | WO | 2017008699 | A1 | 19 January 2017 |
| US | 2018372635 | A1 | 27 December 2018 | WO | 2017015063 | A1 | 26 January 2017 |
| | | | | US | 10712271 | B2 | 14 July 2020 |
| US | 2020116696 | A1 | 16 April 2020 | US | 11782046 | B2 | 10 October 2023 |
| | | | | WO | 2019010234 | A1 | 10 January 2019 |
| CN | 109827928 | A | 31 May 2019 | WO | 2020155716 | A1 | 06 August 2020 |
| | | | | WO | 2020155324 | A1 | 06 August 2020 |

Form PCT/ISA/210 (patent family annex) (July 2022)